# EUROPEAN PATENT APPLICATION

(11) **EP 3 725 897 A1**
(43) Date of publication of application: **21.10.2020**
(21) Application number: 18889432.3
(22) Date of filing: 13.12.2018
(51) Int. Cl.: C12Q 1/6886, C12N 15/113, G01N 33/50

(54) **METHOD FOR ASSISTING DETECTION OF HEAD AND NECK CANCER**

(30) Priority: 13.12.2017 JP 2017238856
(71) Applicant: Hiroshima University, Higashihiroshima-shi Hiroshima 739-8511 (JP)
(72) Inventor: TAHARA, Hidetoshi, Hiroshima-shi, Hiroshima 734-8553 (JP); TAHARA, Makoto, Tokyo 104-0045 (JP)
(74) Representative: Mewburn Ellis LLP
(86) International application number: PCT/JP2018/045994
(87) International publication number: WO 2019/117270

(57) **Abstract**

The present invention aims at providing a method of assisting the detection of head and neck cancer with high accuracy. The present invention provides a method of assisting the detection of head and neck cancer, which includes using as an index the abundance of at least one of miRNAs, isoform miRNAs (isomiRs), precursor miRNAs, transfer RNA fragments (tRFs), or non-coding RNA fragments (LincRNAs or MiscRNAs) contained in a test sample isolated from a living body, whose nucleotide sequence is represented by any one of SEQ ID NOs: 162, 160, 145, 143, 146, 140, 141, 1 to 139, 142, 144, 147 to 159, and 161, wherein a higher abundance of at least one of the miRNAs, isomiRs, precursor miRNAs, transfer RNA fragments, or non-coding RNA fragments whose nucleotide sequence is represented by any one of SEQ ID NOs: 1 to 71 and 118 to 136 than that of healthy subjects or a lower abundance of at least one of the miRNAs, isomiRs, precursor miRNAs, transfer RNA fragments, or non-coding RNA fragments whose nucleotide sequence is represented by any one of SEQ ID NOs: 72 to 117 and 137 to 162 than that of healthy subjects indicates a higher likelihood of having head and neck cancer.

SEQUENCE LISTING

<110>  Hiroshima University

<120>  Method of aiding detection of head and neck cancer

<130>  PF633-PCT

<160>  162

<170>  PatentIn version 3.5

<210>  1
<211>  30
<212>  RNA
<213>  Homo sapiens

<400>  1
gcauuggugg uucaguggua gaauucucgc                                        30


<210>  2
<211>  28
<212>  RNA
<213>  Homo sapiens

<400>  2
cggauagcuc agucgguaga gcaucaga                                          28


<210>  3
<211>  32
<212>  RNA
<213>  Homo sapiens

<400>  3
ucccuggugg ucuagugguu aggauucggc gc                                     32


<210>  4
<211>  31
<212>  RNA
<213>  Homo sapiens

<400>  4
ggcucguugg ucuaggggua ugauucucgg u                                       31


<210>  5
<211>  31
<212>  RNA
<213>  Homo sapiens

<400>  5
gcccggauag cucagucggu agagcaucag a                                      31


<210>  6
<211>  33
<212>  RNA
<213>  Homo sapiens

<400>  6

agcagagugg cgcagcggaa gcgugcuggg ccc                                    33

<210>  7
<211>  31
<212>  RNA
<213>  Homo sapiens

<400>  7
gcccggcuag cucagucggu agagcauggg a                                     31

<210>  8
<211>  31
<212>  RNA
<213>  Homo sapiens

<400>  8
agcagagugg cgcagcggaa gcgugcuggg c                                     31

<210>  9
<211>  21
<212>  RNA
<213>  Homo sapiens

<400>  9
auggcacugg uagaauucac u                                                21

<210>  10
<211>  17
<212>  RNA
<213>  Homo sapiens

<400>  10
ugucaguuug ucaaaua                                                     17

<210>  11
<211>  22
<212>  RNA
<213>  Homo sapiens

<400>  11
ucucccaacc cuuguaccag ug                                               22

<210>  12
<211>  24
<212>  RNA
<213>  Homo sapiens

<400>  12
ugucaguuug ucaaauaccc caag                                             24

<210>  13
<211>  28
<212>  RNA
<213>  Homo sapiens

<400>  13

cggcuagcuc agucgguaga gcauggga                                          28


<210>  14
<211>  23
<212>  RNA
<213>  Homo sapiens

<400>  14
ucucccaacc cuuguaccag ugc                                               23


<210>  15
<211>  19
<212>  RNA
<213>  Homo sapiens

<400>  15
ucucccaacc cuuguacca                                                    19


<210>  16
<211>  30
<212>  RNA
<213>  Homo sapiens

<400>  16
ggcucguugg ucuaggggua ugauucucgc                                        30


<210>  17
<211>  23
<212>  RNA
<213>  Homo sapiens

<400>  17
ugagaacuga auuccauagg cug                                               23


<210>  18
<211>  30
<212>  RNA
<213>  Homo sapiens

<400>  18
agcagagugg cgcagcggaa gcgugcuggg                                        30


<210>  19
<211>  24
<212>  RNA
<213>  Homo sapiens

<400>  19
ucccccaggu gugauucuga uuug                                              24


<210>  20
<211>  21
<212>  RNA
<213>  Homo sapiens

<400>  20

ucaguuuguc aaauacccca a                                              21


<210>  21
<211>  15
<212>  RNA
<213>  Homo sapiens

<400>  21
ugucaguuug ucaaa                                                      15


<210>  22
<211>  16
<212>  RNA
<213>  Homo sapiens

<400>  22
ugucaguuug ucaaau                                                     16


<210>  23
<211>  20
<212>  RNA
<213>  Homo sapiens

<400>  23
ugagaacuga auuccauggg                                                 20


<210>  24
<211>  20
<212>  RNA
<213>  Homo sapiens

<400>  24
ucucccaacc cuuguaccag                                                 20


<210>  25
<211>  18
<212>  RNA
<213>  Homo sapiens

<400>  25
ugucaguuug ucaaauac                                                   18


<210>  26
<211>  22
<212>  RNA
<213>  Homo sapiens

<400>  26
uagcaccauc ugaaaucggu ua                                              22


<210>  27
<211>  20
<212>  RNA
<213>  Homo sapiens

<400>  27

ucaguuuguc aaauacccca                                                20

<210>  28
<211>  23
<212>  RNA
<213>  Homo sapiens

<400>  28
ucccuguccu ccaggagcuc acg                                            23

<210>  29
<211>  23
<212>  RNA
<213>  Homo sapiens

<400>  29
ugucaguuug ucaaauaccc caa                                            23

<210>  30
<211>  22
<212>  RNA
<213>  Homo sapiens

<400>  30
ugagaacuga auuccauagg cu                                             22

<210>  31
<211>  21
<212>  RNA
<213>  Homo sapiens

<400>  31
uaaugccccu aaaaauccuu a                                              21

<210>  32
<211>  22
<212>  RNA
<213>  Homo sapiens

<400>  32
cagugguuuu acccuauggu ag                                             22

<210>  33
<211>  22
<212>  RNA
<213>  Homo sapiens

<400>  33
ugucaguuug ucaaauaccc ca                                             22

<210>  34
<211>  22
<212>  RNA
<213>  Homo sapiens

<400>  34

gucaguuugu caaauacccc aa                                                22

<210>  35
<211>  16
<212>  RNA
<213>  Homo sapiens

<400>  35
gguagcgugg ccgagc                                                        16

<210>  36
<211>  21
<212>  RNA
<213>  Homo sapiens

<400>  36
ucucccaacc cuuguaccag u                                                  21

<210>  37
<211>  24
<212>  RNA
<213>  Homo sapiens

<400>  37
ugagaacuga auuccauagg cugu                                               24

<210>  38
<211>  30
<212>  RNA
<213>  Homo sapiens

<400>  38
ucccuggugg ucuagugguu aggauucggc                                         30

<210>  39
<211>  20
<212>  RNA
<213>  Homo sapiens

<400>  39
ugucaguuug ucaaauaccc                                                    20

<210>  40
<211>  24
<212>  RNA
<213>  Homo sapiens

<400>  40
guccaguuuu cccaggaauc ccuu                                               24

<210>  41
<211>  21
<212>  RNA
<213>  Homo sapiens

<400>  41

caaagaauuc uccuuuuggg c                                                21

<210>  42
<211>  22
<212>  RNA
<213>  Homo sapiens

<400>  42
uaaugccccu aaaaauccuu au                                               22

<210>  43
<211>  23
<212>  RNA
<213>  Homo sapiens

<400>  43
gugucaguuu gucaaauacc cca                                              23

<210>  44
<211>  20
<212>  RNA
<213>  Homo sapiens

<400>  44
ugaccuauga auugacagcc                                                  20

<210>  45
<211>  33
<212>  RNA
<213>  Homo sapiens

<400>  45
gcauuggugg uucaguggua gaauucucgc cug                                   33

<210>  46
<211>  23
<212>  RNA
<213>  Homo sapiens

<400>  46
caaagugcuu acagugcagg uag                                              23

<210>  47
<211>  19
<212>  RNA
<213>  Homo sapiens

<400>  47
ucccuguccu ccaggagcu                                                   19

<210>  48
<211>  21
<212>  RNA
<213>  Homo sapiens

<400>  48

ugucaguuug ucaaauaccc c                                                21

<210>  49
<211>  21
<212>  RNA
<213>  Homo sapiens

<400>  49
gucaguuugu caaauacccc a                                                21

<210>  50
<211>  22
<212>  RNA
<213>  Homo sapiens

<400>  50
uguaaacauc cuacacucuc ag                                               22

<210>  51
<211>  23
<212>  RNA
<213>  Homo sapiens

<400>  51
acucggcgug gcgucggucg ugg                                              23

<210>  52
<211>  22
<212>  RNA
<213>  Homo sapiens

<400>  52
uagcaccauu ugaaaucggu ua                                               22

<210>  53
<211>  20
<212>  RNA
<213>  Homo sapiens

<400>  53
gucaguuugu caaauacccc                                                  20

<210>  54
<211>  24
<212>  RNA
<213>  Homo sapiens

<400>  54
gugucaguuu gucaaauacc ccaa                                             24

<210>  55
<211>  21
<212>  RNA
<213>  Homo sapiens

<400>  55

uguaaacauc cuacacucag c                                                21

<210>  56
<211>  21
<212>  RNA
<213>  Homo sapiens

<400>  56
acuccagccc cacagccuca g                                                21

<210>  57
<211>  21
<212>  RNA
<213>  Homo sapiens

<400>  57
ccuguucucc auuacuuggc u                                                21

<210>  58
<211>  22
<212>  RNA
<213>  Homo sapiens

<400>  58
gcauuggugg uucaguggua ga                                               22

<210>  59
<211>  22
<212>  RNA
<213>  Homo sapiens

<400>  59
cuauacgacc ugcugccuuu cu                                               22

<210>  60
<211>  25
<212>  RNA
<213>  Homo sapiens

<400>  60
gcauuggugg uucaguggua gaauu                                            25

<210>  61
<211>  19
<212>  RNA
<213>  Homo sapiens

<400>  61
uguaaacauc cccgacugg                                                   19

<210>  62
<211>  22
<212>  RNA
<213>  Homo sapiens

<400>  62

cgucaacacu ugcugguuuc cu                                                22

<210>  63
<211>  22
<212>  RNA
<213>  Homo sapiens

<400>  63
aaagugcugu ucgugcaggu ag                                                22

<210>  64
<211>  23
<212>  RNA
<213>  Homo sapiens

<400>  64
uguaaacauc cuugacugga agc                                               23

<210>  65
<211>  16
<212>  RNA
<213>  Homo sapiens

<400>  65
uagcagcacg uaaaua                                                       16

<210>  66
<211>  22
<212>  RNA
<213>  Homo sapiens

<400>  66
ugggucuuug cgggcgagau ga                                                22

<210>  67
<211>  25
<212>  RNA
<213>  Homo sapiens

<400>  67
aaaagcuggg uugagagggc gaaaa                                             25

<210>  68
<211>  21
<212>  RNA
<213>  Homo sapiens

<400>  68
uagcaccauu ugaaaucagu g                                                 21

<210>  69
<211>  22
<212>  RNA
<213>  Homo sapiens

<400>  69

cccauaaagu agaaagcacu ac                                                22

<210>  70
<211>  21
<212>  RNA
<213>  Homo sapiens

<400>  70
cccauaaagu agaaagcacu a                                                 21

<210>  71
<211>  22
<212>  RNA
<213>  Homo sapiens

<400>  71
ugcggggcua gggcuaacag ca                                                22

<210>  72
<211>  21
<212>  RNA
<213>  Homo sapiens

<400>  72
aauacugccu gguaaugaug a                                                 21

<210>  73
<211>  19
<212>  RNA
<213>  Homo sapiens

<400>  73
uucauugcug ucggugggu                                                    19

<210>  74
<211>  18
<212>  RNA
<213>  Homo sapiens

<400>  74
acugucuggu aacgaugu                                                     18

<210>  75
<211>  18
<212>  RNA
<213>  Homo sapiens

<400>  75
ucauugcugu cggugggu                                                     18

<210>  76
<211>  20
<212>  RNA
<213>  Homo sapiens

<400>  76

auucauugcu gucggugggu                                                    20

<210>  77
<211>  22
<212>  RNA
<213>  Homo sapiens

<400>  77
uccgucucag uuacuuuaua gc                                                 22

<210>  78
<211>  21
<212>  RNA
<213>  Homo sapiens

<400>  78
cauucauugc ugucgguggg u                                                  21

<210>  79
<211>  19
<212>  RNA
<213>  Homo sapiens

<400>  79
acuggacuug gagucagga                                                     19

<210>  80
<211>  17
<212>  RNA
<213>  Homo sapiens

<400>  80
cauugcuguc ggugggu                                                       17

<210>  81
<211>  19
<212>  RNA
<213>  Homo sapiens

<400>  81
aguuucccca ggaaucccu                                                     19

<210>  82
<211>  16
<212>  RNA
<213>  Homo sapiens

<400>  82
auugcugucg gugggu                                                        16

<210>  83
<211>  22
<212>  RNA
<213>  Homo sapiens

<400>  83

acauucauug cugucggugg gu                                                22

<210>  84
<211>  18
<212>  RNA
<213>  Homo sapiens

<400>  84
cguuaccauu acugaguu                                                      18

<210>  85
<211>  22
<212>  RNA
<213>  Homo sapiens

<400>  85
agcaccauuu gaaaucagug uu                                                22

<210>  86
<211>  17
<212>  RNA
<213>  Homo sapiens

<400>  86
guuaccauua cugaguu                                                       17

<210>  87
<211>  15
<212>  RNA
<213>  Homo sapiens

<400>  87
uugcugucgg ugggu                                                         15

<210>  88
<211>  17
<212>  RNA
<213>  Homo sapiens

<400>  88
uacaguauag augaugu                                                       17

<210>  89
<211>  18
<212>  RNA
<213>  Homo sapiens

<400>  89
guuaccauua cugaguuu                                                      18

<210>  90
<211>  19
<212>  RNA
<213>  Homo sapiens

<400>  90

accguuacca uuacugagu                                                    19

<210>  91
<211>  22
<212>  RNA
<213>  Homo sapiens

<400>  91
ugagguagga gguuguauag uu                                                 22

<210>  92
<211>  20
<212>  RNA
<213>  Homo sapiens

<400>  92
accaauauua cugugcugcu                                                    20

<210>  93
<211>  25
<212>  RNA
<213>  Homo sapiens

<400>  93
aaaccguuac cauuacugag uuuag                                              25

<210>  94
<211>  23
<212>  RNA
<213>  Homo sapiens

<400>  94
uccuguacug agcugccccg agg                                                23

<210>  95
<211>  20
<212>  RNA
<213>  Homo sapiens

<400>  95
ucguaccgug aguaauaaug                                                    20

<210>  96
<211>  19
<212>  RNA
<213>  Homo sapiens

<400>  96
aauugcacgg uauccaucu                                                     19

<210>  97
<211>  21
<212>  RNA
<213>  Homo sapiens

<400>  97

ugagugugug ugugugagug u                                                21

<210>  98
<211>  22
<212>  RNA
<213>  Homo sapiens

<400>  98
ugagguagua gguugugugg uu                                               22

<210>  99
<211>  20
<212>  RNA
<213>  Homo sapiens

<400>  99
uacaguauag augauguacu                                                  20

<210>  100
<211>  21
<212>  RNA
<213>  Homo sapiens

<400>  100
cacgcucaug cacacaccca c                                                21

<210>  101
<211>  21
<212>  RNA
<213>  Homo sapiens

<400>  101
ugagguagua gguugugugg u                                                21

<210>  102
<211>  19
<212>  RNA
<213>  Homo sapiens

<400>  102
agcagcauug uacagggcu                                                   19

<210>  103
<211>  21
<212>  RNA
<213>  Homo sapiens

<400>  103
cguaccguga guaauaaugc g                                                21

<210>  104
<211>  24
<212>  RNA
<213>  Homo sapiens

<400>  104

gaaaccguua ccauuacuga guuu                                              24

<210>  105
<211>  21
<212>  RNA
<213>  Homo sapiens

<400>  105
uaaagugcug acagugcaga u                                                 21

<210>  106
<211>  22
<212>  RNA
<213>  Homo sapiens

<400>  106
ugagguagua guuugugcug uu                                                22

<210>  107
<211>  15
<212>  RNA
<213>  Homo sapiens

<400>  107
uuaccauuac ugagu                                                        15

<210>  108
<211>  19
<212>  RNA
<213>  Homo sapiens

<400>  108
aaugacacga ucacucccg                                                    19

<210>  109
<211>  20
<212>  RNA
<213>  Homo sapiens

<400>  109
ccaauauuac ugugcugcuu                                                   20

<210>  110
<211>  23
<212>  RNA
<213>  Homo sapiens

<400>  110
ucuacagugc acgugucucc agu                                               23

<210>  111
<211>  23
<212>  RNA
<213>  Homo sapiens

<400>  111

gaaaccguua ccauuacuga guu                                              23

<210>  112
<211>  21
<212>  RNA
<213>  Homo sapiens

<400>  112
uaaggugcau cuagugcaga u                                                21

<210>  113
<211>  22
<212>  RNA
<213>  Homo sapiens

<400>  113
ucguaccgug aguaauaaug cg                                               22

<210>  114
<211>  21
<212>  RNA
<213>  Homo sapiens

<400>  114
ugucuuacuc ccucaggcac a                                                21

<210>  115
<211>  22
<212>  RNA
<213>  Homo sapiens

<400>  115
guaguguuuc cuacuuuaug ga                                               22

<210>  116
<211>  21
<212>  RNA
<213>  Homo sapiens

<400>  116
guaguguuuc cuacuuuaug g                                                21

<210>  117
<211>  23
<212>  RNA
<213>  Homo sapiens

<400>  117
ugagcgccuc gacgacagag ccg                                              23

<210>  118
<211>  22
<212>  RNA
<213>  Homo sapiens

<400>  118

acugcaguga aggcacuugu ag                                              22

<210>  119
<211>  28
<212>  RNA
<213>  Homo sapiens

<400>  119
cccccacugc uaaauuugac uggcuuuu                                        28

<210>  120
<211>  31
<212>  RNA
<213>  Homo sapiens

<400>  120
gcugguccga ugguaguggg uuaucagaac u                                    31

<210>  121
<211>  23
<212>  RNA
<213>  Homo sapiens

<400>  121
agcuacauug ucugcugggu uuc                                             23

<210>  122
<211>  22
<212>  RNA
<213>  Homo sapiens

<400>  122
auauaauaca accugcuaag ug                                              22

<210>  123
<211>  23
<212>  RNA
<213>  Homo sapiens

<400>  123
cagugcaaug uuaaaagggc auu                                             23

<210>  124
<211>  22
<212>  RNA
<213>  Homo sapiens

<400>  124
uuauaaagca augagacuga uu                                              22

<210>  125
<211>  22
<212>  RNA
<213>  Homo sapiens

<400>  125

acaguagucu gcacauuggu ua                                                22

<210>  126
<211>  23
<212>  RNA
<213>  Homo sapiens

<400>  126
aucacauugc cagggauuuc caa                                               23

<210>  127
<211>  23
<212>  RNA
<213>  Homo sapiens

<400>  127
ucaagagcaa uaacgaaaaa ugu                                               23

<210>  128
<211>  22
<212>  RNA
<213>  Homo sapiens

<400>  128
cagugcaaug uuaaaagggc au                                                22

<210>  129
<211>  21
<212>  RNA
<213>  Homo sapiens

<400>  129
uuaugguuug ccugggacug a                                                 21

<210>  130
<211>  26
<212>  RNA
<213>  Homo sapiens

<400>  130
cccccacugc uaaauuugac uggcuu                                            26

<210>  131
<211>  22
<212>  RNA
<213>  Homo sapiens

<400>  131
uucaaguaau ccaggauagg cu                                                22

<210>  132
<211>  32
<212>  RNA
<213>  Homo sapiens

<400>  132

ggcugguccg augguagugg guuaucagaa cu 32

<210> 133
<211> 22
<212> RNA
<213> Homo sapiens

<400> 133
aucacauugc cagggauuuc ca 22

<210> 134
<211> 22
<212> RNA
<213> Homo sapiens

<400> 134
ugagaacuga auuccauggg uu 22

<210> 135
<211> 23
<212> RNA
<213> Homo sapiens

<400> 135
caacggaauc ccaaaagcag cug 23

<210> 136
<211> 31
<212> RNA
<213> Homo sapiens

<400> 136
ggcugguccg augguagugg guuaucagaa c 31

<210> 137
<211> 22
<212> RNA
<213> Homo sapiens

<400> 137
uauugcacuu gucccggccu gu 22

<210> 138
<211> 20
<212> RNA
<213> Homo sapiens

<400> 138
ugagguagua gguugugugg 20

<210> 139
<211> 21
<212> RNA
<213> Homo sapiens

<400> 139

aaaccguuac cauuacugag u                                                21

<210>  140
<211>  23
<212>  RNA
<213>  Homo sapiens

<400>  140
guaaacaucc uugacuggaa gcu                                              23

<210>  141
<211>  21
<212>  RNA
<213>  Homo sapiens

<400>  141
ugagguagua guuuguacag u                                                21

<210>  142
<211>  22
<212>  RNA
<213>  Homo sapiens

<400>  142
uccuguacug agcugccccg ag                                               22

<210>  143
<211>  20
<212>  RNA
<213>  Homo sapiens

<400>  143
uagcagcacg uaaauauugg                                                  20

<210>  144
<211>  20
<212>  RNA
<213>  Homo sapiens

<400>  144
aaaccguuac cauuacugag                                                  20

<210>  145
<211>  21
<212>  RNA
<213>  Homo sapiens

<400>  145
uggagagaaa ggcaguuccu g                                                21

<210>  146
<211>  20
<212>  RNA
<213>  Homo sapiens

<400>  146

ugagguagua gguuguauag                                                    20


<210>  147
<211>  21
<212>  RNA
<213>  Homo sapiens

<400>  147
uauugcacuu gucccggccu g                                                  21


<210>  148
<211>  21
<212>  RNA
<213>  Homo sapiens

<400>  148
cauugcacuu gucucggucu g                                                  21


<210>  149
<211>  21
<212>  RNA
<213>  Homo sapiens

<400>  149
accaauauua cugugcugcu u                                                  21


<210>  150
<211>  20
<212>  RNA
<213>  Homo sapiens

<400>  150
ugagguagua gauuguauag                                                    20


<210>  151
<211>  20
<212>  RNA
<213>  Homo sapiens

<400>  151
cauugcacuu gucucggucu                                                    20


<210>  152
<211>  21
<212>  RNA
<213>  Homo sapiens

<400>  152
aaugacacga ucacucccgu u                                                  21


<210>  153
<211>  21
<212>  RNA
<213>  Homo sapiens

<400>  153

ugaggggcag agagcgagac u                                                21

<210>  154
<211>  21
<212>  RNA
<213>  Homo sapiens

<400>  154
ucaggcucag uccccucccg a                                                21

<210>  155
<211>  21
<212>  RNA
<213>  Homo sapiens

<400>  155
uccuguacug agcugccccg a                                                21

<210>  156
<211>  20
<212>  RNA
<213>  Homo sapiens

<400>  156
uccuguacug agcugccccg                                                  20

<210>  157
<211>  21
<212>  RNA
<213>  Homo sapiens

<400>  157
ugagguagua guuugugcug u                                                21

<210>  158
<211>  20
<212>  RNA
<213>  Homo sapiens

<400>  158
agagguagua gguugcauag                                                  20

<210>  159
<211>  17
<212>  RNA
<213>  Homo sapiens

<400>  159
uccuguacug agcugcc                                                     17

<210>  160
<211>  20
<212>  RNA
<213>  Homo sapiens

<400>  160

ugagguagua guuugugcug                                                20

<210>  161
<211>  20
<212>  RNA
<213>  Homo sapiens

<400>  161
ucaggcucag uccccucccg                                                20

<210>  162
<211>  15
<212>  RNA
<213>  Homo sapiens

<400>  162
ucauguauga ugcug                                                     15

## Description

### TECHNICAL FIELD

The present invention relates to a method of assisting the detection of head and neck cancer.

### BACKGROUND ART

Head and neck cancer refers to cancer that occurs in a body region below the brain and above the clavicles. Vital functions such as breathing and eating, and socially important daily life functions such as speaking, tasting, and hearing are predominantly related to the head and neck region. Thus, a therapy to treat cancer while keeping the balance between curability and QOL is needed because any lesion in the head and neck region may directly affect QOL. Additionally, aesthetic considerations are also necessary because the head and neck region is involved in maintenance of facial morphology and/or in expression of feelings.

As methods to detect such cancer including head and neck cancer, methods in which the abundance of microRNA (hereinafter referred to as "miRNA") in blood is used as an index are proposed (Patent Documents 1 to 5).

### PRIOR ART DOCUMENTS

### Patent Documents

Patent Document 1 WO 2009/133915
Patent Document 2 WO 2012/161124
Patent Document 3 JP 2013-539018 T
Patent Document 4 JP 2015-502176 T
Patent Document 5 JP 2015-51011 A

### SUMMARY OF THE INVENTION

### PROBLEM TO BE SOLVED BY THE INVENTION

As described above, various miRNAs have been proposed as indexes for the detection of cancer including head and neck cancer and, needless to say, it is advantageous if head and neck cancer can be detected with higher accuracy.

Thus, an object of the present invention is to provide a method of assisting the detection of head and neck cancer which assists in highly accurate detection of head and neck cancer.

### MEANS FOR SOLVING THE PROBLEM

As a result of intensive study, the inventors newly found miRNAs, isoform miRNAs (isomiRs), precursor miRNAs, transfer RNA fragments (tRFs), and non-coding RNA fragments (LincRNAs, MiscRNAs) which increase or decrease in abundance in head and neck cancer, and discovered that use of those RNA molecules as indexes enables highly accurate detection of head and neck cancer, and thereby completed the present invention.

That is, the present invention provides the followings.
(1) A method of assisting the detection of head and neck cancer, using as an index the abundance of at least one of miRNAs, isoform miRNAs (isomiRs), precursor miRNAs, transfer RNA fragments (tRFs), or non-coding RNA fragments (LincRNAs or MiscRNAs) contained in a test sample isolated from a living body, whose nucleotide sequence is represented by any one of SEQ ID NOs: 162, 160, 145, 143, 146, 140, 141, 1 to 139, 142, 144, 147 to 159, and 161, wherein a higher abundance of at least one of the miRNAs, isomiRs, precursor miRNAs, transfer RNA fragments, or non-coding RNA fragments whose nucleotide sequence is represented by any one of SEQ ID NOs: 1 to 71 and 118 to 136 than that of healthy subjects or a lower abundance of at least one of the miRNAs, isomiRs, precursor miRNAs, transfer RNA fragments, or non-coding RNA fragments whose nucleotide sequence is represented by any one of SEQ ID NOs: 72 to 117 and 137 to 162 than that of healthy subjects indicates a higher likelihood of having head and neck cancer.
(2) The method according to (1), wherein the abundance of at least one of miRNAs, isoform miRNAs (isomiRs), precursor miRNAs, transfer RNA fragments (tRFs), or non-coding RNA fragments (LincRNAs or MiscRNAs) whose nucleotide sequence is represented by any one of SEQ ID NOs: 162, 160, 145, 143, 146, 140, and 141 is used as an index.
(3) The method according to (1), wherein the abundance of at least one of miRNAs, isoform miRNAs (isomiRs), precursor miRNAs, or transfer RNA fragments whose nucleotide sequence is represented by any one of SEQ ID NOs: 92, 2, 74, 73, 75, 84, 32, 77, 18, 1, 3 to 31, 33 to 72, 76, 78 to 83, 85 to 91, and 93 to 116 is used as an index.
(4) The method according to (3), wherein the abundance of an isomiR whose nucleotide sequence is represented by SEQ ID NO: 115 or 116 is used as an index.
(5) The method according to (3) or (4), wherein the abundance of a miRNA whose nucleotide sequence is represented by SEQ ID NO: 11 and a miRNA whose nucleotide sequence is represented by SEQ ID NO: 30 is used as an index.
(6) The method according to (3) or (4), wherein the abundance of a miRNA whose nucleotide sequence is represented by SEQ ID NO: 11 and a miRNA whose nucleotide sequence is represented by SEQ ID NO: 26 is used as an index.
(7) The method according to (3) or (4), wherein the abundance of a miRNA whose nucleotide sequence is represented by SEQ ID NO: 11 and a miRNA whose nucleotide sequence is represented by SEQ ID NO: 117 is used as an index.
(8) The method according to (3) or (4), wherein the abundance of a miRNA whose nucleotide sequence is represented by SEQ ID NO: 30 and a miRNA whose nucleotide sequence is represented by SEQ ID NO: 118 is used as an index.
(9) The method according to (1), wherein the abundance of a miRNA whose nucleotide sequence is represented by SEQ ID NO: 157 and a miRNA whose nucleotide sequence is represented by SEQ ID NO: 162 is used as an index.
(10) The method according to any one of (3) to (8), wherein the head and neck cancer is tongue cancer.

### EFFECT OF THE INVENTION

By the method of the present invention, head and neck cancer can be highly accurately and yet conveniently detected. Thus, the method of the present invention will greatly contribute to the detection of head and neck cancer.

### MODE FOR CARRYING OUT THE INVENTION

As described above, the abundance of a specified miRNAs, isomiRs, precursor miRNAs, transfer RNA fragments (tRFs), or non-coding RNA fragments (LincRNAs or MiscRNA) (hereinafter sometimes referred to as "miRNAs or the like" for convenience) contained in a test sample isolated from a living body is used as an index in the method of the present invention. The nucleotide sequence of these miRNAs or the like themselves are as shown in Sequence Listing. The list of miRNAs or the like used in the method of the present invention is presented in Tables 1-1 to 1-7 below.

**Table 1-1**

| SEQ ID NO: | Class | Archetype | Type | Length (nucleotides) | Sequence |
|---|---|---|---|---|---|
| 1 | tRF | tRNA-Gly-CCC-1-1//··· *1 | Exact | 30 | gcauuggugguucagugguagaauucucgc |
| 2 | tRF | tRNA-Lys-TTT-3-1//··· *2 | Exact | 28 | cggauagcucagucgguagagcaucaga |
| 3 | tRF | tRNA-Glu-CTC-1-1//··· *3 | Exact | 32 | ucccugguggucuagugguuaggauucggcgc |
| 4 | tRF | tRNA-Pro-TGG-2-1 | Exact | 31 | ggcucguuggucuagggguaugauucucggu |
| 5 | tRF | tRNA-Lys-TTT-3-1//··· *4 | Exact | 31 | gcccggauagcucagucgguagagcaucaga |
| 6 | tRF | tRNA-iMet-CAT-1-1//··· *5 | Exact | 33 | agcagaguggcgcagcggaagcgugcugggccc |
| 7 | tRF | tR-NA-Ls-CTT-1-1//··· *6 | Exact | 31 | gcccggcuagcucagucgguagagcauggga |
| 8 | tRF | tRNA-iMet-CAT-1-1//··· *7 | Exact | 31 | agcagaguggcgcagcggaagcgugcugggc |
| 9 | isomiR | mir-183 | Mature 5' sub | 21 | auggcacugguagaauucacu |
| 10 | isomiR | mir-223 | Mature 3' sub | 17 | ugucaguuugucaaaua |
| 11 | miRNA | mir-150 | Mature 5' | 22 | ucucccaacccuuguaccagug |
| 12 | isomiR | mir-223 | Mature 3' super | 24 | ugucaguuugucaaauaccccaag |
| 13 | tRF | tRNA-Lys-CTT-1-1//··· *8 | Exact | 28 | cggcuagcucagucgguagagcauggga |
| 14 | isomiR | mir-150 | Mature 5' super | 23 | ucucccaacccuuguaccagugc |
| 15 | isomiR | mir-150 | Mature 5' sub | 19 | ucucccaacccuuguacca |
| 16 | tRF | tRNA-Pro-AGG-1-1//··· *9 | Exact | 30 | ggcucguuggucuagggguaugauucucgc |
| 17 | isomiR | mir-146b | Mature 5' super | 23 | ugagaacugaauuccauaggcug |
| 18 | tRF | tRNA-iMet-CAT-1-1//··· *10 | Exact | 30 | agcagaguggcgcagcggaagcgugcuggg |
| 19 | isomiR | mir-361 | Mature 3' super | 24 | ucccccaggugugauucugauuug |
| 20 | isomiR | mir-223 | Mature 3' sub/super | 21 | ucaguuugucaaauaccccaa |
| 21 | precursor | mir-223 | precursor miRNA | 15 | ugucaguuugucaaa |
| 22 | precursor | mir-223 | precursor miRNA | 16 | ugucaguuugucaaau |
| 23 | isomiR | mir-146a | Mature 5' sub | 20 | ugagaacugaauuccauggg |
| 24 | isomiR | mir-150 | Mature 5' sub | 20 | ucucccaacccuuguaccag |
| 25 | isomiR | mir-223 | Mature 3' sub | 18 | ugucaguuugucaaauac |
| 26 | miRNA | mir-29a | Mature 3' | 22 | uagcaccaucugaaaucgguua |
| 27 | isomiR | mir-223 | Mature 3' sub | 20 | ucaguuugucaaauacccca |
| 28 | miRNA | mir-339 | Mature 5' | 23 | ucccuguccuccaggagcucacg |

**Table 1-2**

| SEQ ID NO: | Class | Archetype | Type | Length (nucleotides) | Sequence |
|---|---|---|---|---|---|
| 29 | isomiR | mir-223 | Mature 3' super | 23 | ugucaguuugucaaauaccccaa |
| 30 | miRNA | mir-146b | Mature 5' | 22 | ugagaacugaauuccauaggcu |
| 31 | isomiR | mir-365a//mir-365b | Mature 3' sub | 21 | uaaugccccuaaaaauccuua |
| 32 | miRNA | mir-140 | Mature 5' | 22 | cagugguuuuacccuaugguag |
| 33 | miRNA | mir-223 | Mature 3' | 22 | ugucaguuugucaaauacccca |
| 34 | isomiR | mir-223 | Mature 3' sub/super | 22 | gucaguuugucaaauaccccaa |
| 35 | tRF | tRNA-Leu-AAG-1-1//··· *11 | Exact | 16 | gguagcguggccgagc |
| 36 | isomiR | mir-150 | Mature 5' sub | 21 | ucucccaacccuuguaccagu |
| 37 | isomiR | mir-146b | Mature 5' super | 24 | ugagaacugaauuccauaggcugu |
| 38 | tRF | tRNA-Glu-CTC-1-1//··· *12 | Exact | 30 | ucccugguggucuagugguuaggauucggc |
| 39 | isomiR | mir-223 | Mature 3' sub | 20 | ugucaguuugucaaauaccc |
| 40 | isomiR | mir-145 | Mature 5' super | 24 | guccaguuuucccaggaaucccuu |
| 41 | isomiR | mir-186 | Mature 5' sub | 21 | caaagaauucuccuuuugggc |
| 42 | miRNA | mir-365a//mir-365b | Mature 3' | 22 | uaaugccccuaaaaauccuuau |
| 43 | isomiR | mir-223 | Mature 3' super | 23 | gugucaguuugucaaauacccca |
| 44 | isomiR | mir-192 | Mature 5' sub | 20 | ugaccuaugaauugacagcc |
| 45 | tRF | tRNA-Gly-GCC-2-1//··· *13 | Exact | 33 | gcauuggugguucagugguagaauucucgccug |
| 46 | miRNA | mir-17 | Mature 5' | 23 | caaagugcuuacagugcagguag |
| 47 | isomiR | mir-339 | Mature 5' sub | 19 | ucccuguccuccaggagcu |
| 48 | isomiR | mir-223 | Mature 3' sub | 21 | ugucaguuugucaaauacccc |
| 49 | isomiR | mir-223 | Mature 3' sub | 21 | gucaguuugucaaauacccca |
| 50 | isomiR | mir-30c-2//mir-30c-1 | Mature 5' sub | 22 | uguaaacauccuacacucucag |
| 51 | isomiR | mir-1307 | Mature 3' super | 23 | acucggcguggcgucggucgugg |
| 52 | miRNA | mir-29c | Mature 3' | 22 | uagcaccauuugaaaucgguua |
| 53 | isomiR | mir-223 | Mature 3' sub | 20 | gucaguuugucaaauacccc |
| 54 | isomiR | mir-223 | Mature 3' super | 24 | gugucaguuugucaaauaccccaa |
| 55 | isomiR | mir-30b | Mature 5' sub | 21 | uguaaacauccuacacucagc |
| 56 | isomiR | mir-766 | Mature 3' sub | 21 | acuccagccccacagccucag |
| 57 | isomiR | mir-26b | Mature 3' sub | 21 | ccuguucuccauuacuuggcu |

**Table 1-3**

| SEQ ID NO: | Class | Archetype | Type | Length (nucleotides) | Sequence |
|---|---|---|---|---|---|
| 58 | tRF | tRNA-Gly-CCC-1-1//··· *14 | Exact | 22 | gcauuggugguucagugguaga |
| 59 | miRNA | let-7d | Mature 3' | 22 | cuauacgaccugcugccuuucu |
| 60 | tRF | tRNA-Gly-CCC-1-1//··· *15 | Exact | 25 | gcauuggugguucagugguagaauu |
| 61 | isomiR | mir-30d | Mature 5' sub | 19 | uguaaacauccccgacugg |
| 62 | miRNA | mir-505 | Mature 3' | 22 | cgucaacacuugcugguuuccu |
| 63 | isomiR | mir-93 | Mature 5' sub | 22 | aaagugcuguucgugcagguag |
| 64 | isomiR | mir-30e | Mature 5' super | 23 | uguaaacauccuugacuggaagc |
| 65 | precursor | mir-16-1//mir-16-2 | precursor miRNA | 16 | uagcagcacguaaaua |
| 66 | miRNA | mir-193a | Mature 5' | 22 | ugggucuuugcgggcgagauga |
| 67 | isomiR | mir-320a | Mature 3' super | 25 | aaaagcuggguugagagggcgaaaa |
| 68 | isomiR | mir-29b-1//mir-29b-2 | Mature 3' sub | 21 | uagcaccauuugaaaucagug |
| 69 | isomiR | mir-142 | Mature 5' sub/super | 22 | cccauaaaguagaaagcacuac |
| 70 | isomiR | mir-142 | Mature 5' sub/super | 21 | cccauaaaguagaaagcacua |
| 71 | miRNA | mir-744 | Mature 5' | 22 | ugcggggcuagggcuaacagca |
| 72 | isomiR | mir-200b | Mature 3' sub | 21 | aauacugccugguaaugauga |
| 73 | isomiR | mir-181b-1//mir-181b-2 | Mature 5' sub | 19 | uucauugcugucggugggu |
| 74 | isomiR | mir-200a | Mature 3' sub | 18 | acugucugguaacgaugu |
| 75 | isomiR | mir-181b-1//mir-181b-2 | Mature 5' sub | 18 | ucauugcugucggugggu |
| 76 | isomiR | mir-181b-1//mir-81b-2 | Mature 5' sub | 20 | auucauugcugucggugggu |
| 77 | miRNA | mir-340 | Mature 3' | 22 | uccgucucaguuacuuuauagc |
| 78 | isomiR | mir-181b-1//mir-181b-2 | Mature 5' sub | 21 | cauucauugcugucggugggu |
| 79 | miRNA | mir-378e | Mature 3' | 19 | acuggacuuggagucagga |
| 80 | precursor | mir-181b-1//mir-181b-2 | precursor miRNA | 17 | cauugcugucggugggu |
| 81 | isomiR | mir-145 | Mature 5' sub | 19 | aguuuucccaggaaucccu |
| 82 | precursor | mir-181b-1//mir-181b-2 | precursor miRNA | 16 | auugcugucggugggu |
| 83 | isomiR | mir-181b-1//mir-181b-2 | Mature 5' sub | 22 | acauucauugcugucggugggu |
| 84 | isomiR | mir-451 a | Mature 5' sub | 18 | cguuaccauuacugaguu |
| 85 | isomiR | mir-29h-1//mir-29b-2 | Mature 3' sub | 22 | agcaccauuugaaaucaguguu |

**Table 1-4**

| SEQ ID NO: | Class | Archetype | Type | Length (nucleotides) | Sequence |
|---|---|---|---|---|---|
| 86 | isomiR | mir-451a | Mature 5' sub | 17 | guuaccauuacugaguu |
| 87 | precursor | mir-181b-1//mir-181b-2 | precursor miRNA | 15 | uugcugucggugggu |
| 88 | isomiR | mir-144 | Mature 3' sub | 17 | uacaguauagaugaugu |
| 89 | isomiR | mir-451 a | Mature 5' sub/super | 18 | guuaccauuacugaguuu |
| 90 | isomiR | mir-451a | Mature 5' sub | 19 | accguuaccauuacugagu |
| 91 | miRNA | let-7e | Mature 5' | 22 | ugagguaggagguuguauaguu |
| 92 | isomiR | mir-16-2 | Mature 3' sub/super | 20 | accaauauuacugugcugcu |
| 93 | isomiR | mir-451 a | Mature 5' super | 25 | aaaccguuaccauuacugaguuuag |
| 94 | isomiR | mir-486-1 | Mature 5' super | 23 | uccuguacugagcugccccgagg |
| 95 | isomiR | mir-126 | Mature 3' sub | 20 | ucguaccgugaguaauaaug |
| 96 | isomiR | mir-363 | Mature 3' sub | 19 | aauugcacgguauccaucu |
| 97 | isomiR | mir-574 | Mature 5' sub | 21 | ugaguguguguffugugagugu |
| 98 | miRNA | let-7b | Mature 5' | 22 | ugagguaguagguugugugguu |
| 99 | miRNA | mir-144 | Mature 3' | 20 | uacaguauagaugauguacu |
| 100 | isomiR | mir-574 | Mature 3' sub | 21 | cacgcucaugcacacacccac |
| 101 | isomiR | let-7b | Mature 5' sub | 21 | ugagguaguagguuguguggu |
| 102 | isomiR | mir-103a-2//mir-103a-1//mir-107 | Mature 3' sub | 19 | agcagcauuguacagggcu |
| 103 | isomiR | mir-126 | Mature 3' sub | 21 | cguaccgugaguaauaaugcg |
| 104 | isomiR | mir-451a | Mature 5' super | 24 | gaaaccguuaccauuacugaguuu |
| 105 | miRNA | mir-106b | Mature 5' | 21 | uaaagugcugacagugcagau |
| 106 | miRNA | let-7i | Mature 5' | 22 | ugagguaguaguuugugcuguu |
| 107 | precursor | mir-45 1a | precursor miRNA | 15 | uuaccauuacugagu |
| 108 | isomiR | mir-425 | Mature 5' sub | 19 | aaugacacgaucacucccg |
| 109 | isomiR | mir-16-2 | Mature 3' sub | 20 | ccaauauuacugugcugcuu |
| 110 | miRNA | mir-139 | Mature 5' | 23 | ucuacagugcacgugucuccagu |
| 111 | isomiR | mir-451 a | Mature 5' super | 23 | gaaaccguuaccauuacugagu u |
| 112 | isomiR | mir-18a | Mature 5' sub | 21 | uaaggugcaucuagugcagau |
| 113 | miRNA | mir-126 | Mature 3' | 22 | ucguaccsugaguaauaaugcg |

**Table 1-5**

| SEQ ID NO: | Class | Archetype | Type | Length (nucleotides) | Sequence |
|---|---|---|---|---|---|
| 114 | isomiR | mir-550a-1//mir-550a-2//mir-550a-3 | Mature 3' sub | 21 | ugucuuacucccucaggcaca |
| 115 | isomiR | mir-142 | Mature 3' sub | 22 | guaguguuuccuacuuuaugga |
| 116 | isomiR | mir-142 | Mature 3' sub | 21 | guaguguuuccuacuuuaugg |
| 117 | miRNA | mir-339 | Mature 3' | 23 | ugagcgccucgacgacagagccg |
| 118 | miRNA | mir-17 | Mature 3' | 22 | acugcagugaaggcacuuguag |
| 119 | MiscRNA | ENST00000363745.1// ··· *16 | Exact | 28 | cccccacugcuaaauuugacuggcuuuu |
| 120 | MiscRNA | ENST00000364600.1//··· *17 | Exact | 31 | gcugguccgaugguaguggguuaucagaacu |
| 121 | miRNA | mir-221 | Mature 3' | 23 | agcuacauugucugcuggguuuc |
| 122 | miRNA | mir-374b | Mature 5' | 22 | auauaauacaaccugcuaagug |
| 123 | isomiR | mir-130a | Mature 3' super | 23 | cagugcaauguuaaaagggcauu |
| 124 | miRNA | mir-340 | Mature 5' | 22 | uuauaaagcaaugagacugauu |
| 125 | miRNA | mir-199a-1//mir-199a-2//mir-199b | Mature 3' | 22 | acaguagucugcacauugguua |
| 126 | isomiR | mir-23a | Mature 3' super | 23 | aucacauugccagggauuuccaa |
| 127 | miRNA | mir-335 | Mature 5' | 23 | ucaagagcaauaacgaaaaaugu |
| 128 | miRNA | mir-130a | Mature 3' | 22 | cagugcaauguuaaaagggcau |
| 129 | isomiR | mir-584 | Mature 5' sub | 21 | uuaugguuugccugggacuga |
| 130 | MiscRNA | ENST00000363745.1//··· *18 | Exact | 26 | cccccacugcuaaauuugacuggcuu |
| 131 | miRNA | mir-26a-1//mir-26a-2 | Mature 5' | 22 | uucaaguaauccaggauaggcu |
| 132 | MiscRNA | ENST00000364600.1//··· *17 | Exact | 32 | ggcugguccgaugguaguggguuaucagaacu |
| 133 | isomiR | mir-23a | Mature 3' super | 22 | aucacauugccagggauuucca |
| 134 | miRNA | mir-146a | Mature 5' | 22 | ugagaacugaauuccauggguu |
| 135 | miRNA | mir-191 | Mature 5' | 23 | caacggaaucccaaaagcagcug |
| 136 | MiscRNA | ENST00000364600.1//··· *17 | Exact | 31 | ggcugguccgaugguaguggguuaucagaac |
| 137 | miRNA | mir-92a-1//mir-92a-2 | Mature 3' | 22 | uauugcacuugucccggccugu |
| 138 | isomiR | let-7b | Mature 5' sub | 20 | ugagguaguagguugugugg |
| 139 | isomiR | mir-451 a | Mature 5' sub | 21 | aaaccguuaccauuacugagu |
| 140 | isomiR | mir-30e | Mature 5' sub/super | 23 | guaaacauccuugacuggaagcu |
| 141 | isomiR | let-7g | Mature 5' sub | 21 | ugagguaguaguuuguacagu |
| 142 | miRNA | mir-486-1//mir-486-2 | Mature 5' | 22 | uccuguacugagcugccccgag |

**Table 1-6**

| SEQ ID NO: | Class | Archetype | Type | Length (nucleotides) | Sequence |
|---|---|---|---|---|---|
| 143 | isomiR | mir-16-11/mir-16-2 | Mature 5' sub | 20 | uagcagcacguaaauauugg |
| 144 | isomiR | mir-451a | Mature 5' sub | 20 | aaaccguuaccauuacugag |
| 145 | isomiR | mir-185 | Mature 5' sub | 21 | uggagagaaaggcaguuccug |
| 146 | isomiR | let-7a-1//let-7a-2//let-7a-3 | Mature 5' sub | 20 | ugagguaguagguuguauag |
| 147 | isomiR | mir-92a-1//mir-92a-2 | Mature 3' sub | 21 | ugagguaguagguuguauag |
| 148 | isomiR | mir-25 | Mature 3' sub | 21 | cauugcacuugucucggucug |
| 149 | isomiR | mir-16-2 | Mature 3' sub/super Mature 3' sub/super | 21 | accaauauuacugucugcuu |
| 150 | isomiR | let-7f-1//let-7f-2 | Mature 5' sub | 20 | ugagguaguagauuguauag |
| 151 | isomiR | mir-25 | Mature 3' sub | 20 | cauugcacuugucucggucu |
| 152 | isomiR | mir-425 | Mature 5' sub | 21 | aaugacacgucacucccguu |
| 153 | isomiR | mir-423 | Mature 5' sub | 21 | ugaggggcagagagcgagacu |
| 154 | isomiR | mir-484 | Mature 5' sub | 21 | ucaggcucaguccccucccga |
| 155 | isomiR | mir-486-1//mir-486-2 | Mature 5' sub | 21 | uccuguacugagcugccccga |
| 156 | isomiR | mir-486-1//mir-486-2 | Mature 5' sub | 20 | uccuguacugagcugccccg |
| 157 | isomiR | let-7i | Mature 5' sub | 21 | ugagguaguaguuugugcugu |
| 158 | isomiR | let-7d | Mature 5' sub | 20 | agagguagguugcauag |
| 159 | isomiR | mir-486-1//mir-486-2 | Mature 5' sub | 17 | uccuguacugagcugcc |
| 160 | isomiR | let-7i | Mature 5' sub | 20 | ugagguaguaguuugugcug |
| 161 | isomiR | mir-484 | Mature 5' sub | 20 | ucaggcucaguccccucccg |
| 162 | LincRNA | ENST00000627566.1 | Exact | 15 | ucauguaugaugcug |
| *1:tRNA-Gly-CCC-1-1//tRNA-Gly-CCC-1-2//tRNA-Gly-GCC-2-1//tRNA-Gly-GCC-2-2//tRNA-Gly-GCC-2-3//tRNA-Gly-GCC-2-4//tRNA-Gly-GCC-2-5 //tRNA-Gly-GCC-2-6//tRNA-Gly-GCC-3-1//tRNA-Gly-GCC-5-1 | | | | | |
| *2:tRNA-Lys-TTT-3-1//tRNA-Lys-TTT-3-2//tRNA-Lys-TTT-3-3//tRNA-Lys-TTT-3-4//tRNA-Lys-TTT-3-5//tRNA-Lys-TTT-5-1 | | | | | |
| *3:tRNA-Glu-CTC-1-1//tRNA-Glu-CTC-1-2//tRNA-Glu-CTC-1-3//tRNA-Glu-CTC-1-4//tRNA-Glu-CTC-1-5//tRNA-Glu-CTC-1-6//tRNA-Glu-CTC-1-7//t RNA-Glu-CTC-2-1 | | | | | |
| *4:tRNA-Lys-TTT-3-1//tRNA-Lys-TT-3-2//tRNA-Lys-TTT-3-3//tRNA-Lys-TTT-3-4//tRNA-Lys-TTT3-5//tRNA-Lys-TTT-5-1 | | | | | |
| *5:tRNA-iMet-CAT-1-1//tRNA-iMet-CAT-1-2//tRNA-iMet-CAT-1-3//tRNA-iMet-CAT-1-4//tRNA-iMet-CAT-1-5//tRNA-iMet-CAT-1-6//tRNA-iMet-CAT-1-7//tRNA-iMet-CAT-1-8//tRNA-iMet-CAT-2-1 | | | | | |

**Table 1-7**

| |
|---|
| *6:tRNA-Lys-CTT-1-1//tRNA-Lys-CTT-1-2//tRNA-Lys-CTT-4-1 |
| *7:tRNA-iMet-CAT-1-1//tRNA-iMet-CAT-1-2//tRNA-iMet-CAT-1-3//tRNA-iMet-CAT-1-4//tRNA-iMet-CAT-1-5//tRNA-iMet-CAT-1-6//tRNA-iMet-CAT-1-7//tRNA-iMet-CAT-1-8//tRNA-iMet-CAT-2-1 |
| *8:tRNA-Lys-CTT-1-1//tRNA-Lys-CTT-1-2//tRNA-Lys-CTT-4-1 |
| *9:tRNA-Pro-AGG-1-1//tRNA-Pro-AGG-2-1//tRNA-Pro-AGG-2-2//tRNA-Pro-AGG-2-3//tRNA-Pro-AGG-2-41/tRNA-Pro-AGG-2-5//tRNA-Pro-AGG-2-6 //tRNA-Pro-AGG-2-7//tRNA-Pro-AGG-2-8//tRNA-Pro-CGG-1-1//tRNA-Pro-CGG-1-2//tRNA-Pro-CGG-1-3//tRNA-Pro-CGG-2-1//tRNA-Pro-TGG-3-1//t RNA-Pro-TGG-3-2//tRNA-Pro-TGG-3-3//tRNA-Pro-TGG-3-4//tRNA-Pro-TGG-3-5 |
| *10:tRNA-iMet-CAT-1-1//tRNA-iMet-CAT-1-2//tRNA-iMet-CAT-1-3//tRNA-iMet-CAT-1-4//tRNA-iMet-CAT-1-5//tRNA-iMet-CAT-1-6//tRNA-iMet-CAT -1-7//tRNA-iMet-CAT-1-8//tRNA-iMet-CAT-2-1 |
| *11:tRNA-Leu-AAG-1-1//tRNA-Leu-AAG-1-2//tRNA-Leu-AAG-1-3//tRNA-Leu-AAG-2-1//tRNA-Leu-AAG-2-2//tRNA-Leu-AAG-2-3//tRNA-Leu-AAG -2-4//tRNA-Leu-AAG-3-1//tRNA-Leu-TAG-1-1 |
| *12:tRNA-Glu-CTC-1-1//tRNA-Glu-CTC-1-2//tRNA-Glu-CTC-1-3//tRNA-Glu-CTC-1-4//tRNA-Glu-CTC-1-5//tRNA-Glu-CTC-1-6//tRNA-Glu-CTC-1-7/ /tRNA-Glu-CTC-2-1 |
| *13:tRNA-Gly-GCC-2-1//tRNA-Gly-GCC-2-2//tRNA-Gly-GCC-2-3//tRNA-Gly-GCC-2-4//tRNA-Gly-GCC-2-5//tRNA-Gly-GCC-2-6//tRNA-Gly-GCC-3-1//tRNA-Gly-GCC-5-1 |
| *14:tRNA-Gly-CCC-1-1//tRNA-Gly-CCC-1-2//tRNA-Gly-GCC-2-1//tRNA-Gly-GCC-2-2//tRNA-Gly-GCC-2-3//tRNA-Gly-GCC-2-4//tRNA-Gly-GCC-2-5//tRNA-Gly-GCC-2-6//tRNA-Gly-GCC-3-1//tRNA-Gly-GCC-5-1 |
| *15:tRNA-Gly-CCC-1-1//tRNA-Gly-CCC-1-2//tRNA-Gly-GCC-2-1//tRNA-Gly-GCC-2-2//tRNA-Gly-GCC-2-3//tRNA-Gly-GCC-2-4//tRNA-Gly-GCC-2-5//tRNA-Gly-GCC-2-6//tRNA-Gly-GCC-3-1//tRNA-Gly-GCC-5-1 |
| * 16:ENST00000363745.1//ENST00000516507.1 |
| *17 :ENST00000364600.1//ENST00000577883.2//ENST00000577984.2//ENST00000516507.1//ENST00000481041.3//ENST00000579625.2//ENST00000 365571.2//ENST00000578877.2//ENST00000364908.1 |
| *18:ENST00000363745.1//ENST00000364409.1//ENST00000516507.1 //ENST00000391107.1//ENST00000459254.1 |

Among those miRNAs or the like, miRNAs or the like whose nucleotide sequences are represented by SEQ ID NOs: 1 to 162 (for example, "a miRNA or the like whose nucleotide sequence is represented by SEQ ID NO: 1" is hereinafter sometimes referred to simply as "a miRNA or the like represented by SEQ ID NO: 1" or "one represented by SEQ ID NO: 1" for convenience) are present in serum or exosomes.

In many of those miRNAs or the like, the logarithm of the ratio of the abundance in serum or exosomes from patients with head and neck cancer to the abundance in serum or exosomes from healthy subjects (represented by "log FC" which means the logarithm of FC (fold change) to base 2) is not less than 1.00 in absolute value, showing a statistical significance (*t*-test; *p* < 0.05).

The abundance of miRNAs or the like represented by SEQ ID NOs: 1 to 71 and 118 to 136 is higher in patients with head and neck cancer than in healthy subjects, while the abundance of miRNAs or the like represented by SEQ ID NOs: 72 to 117 and 137 to 162 is lower in patients with head and neck cancer than in healthy subjects.

By a method in which among those, any of the combinations of the miRNAs represented by SEQ ID NOs: 11 and 30, SEQ ID NOs: 11 and 26, SEQ ID NOs: 11 and 117, SEQ ID NOs: 30 and 118, and SEQ ID NOs: 157 and 162 is used as an index, even early tongue cancer can be detected, as specifically described in Examples below.

The accuracy of each cancer marker is indicated using the area under the ROC curve (AUC: Area Under Curve) as an index, and cancer markers with an AUC value of 0.7 or higher are generally considered effective. AUC values of 0.90 or higher, 0.97 or higher, 0.99 or higher, and 1.00 correspond to cancer markers with high accuracy, very high accuracy, quite high accuracy, and complete accuracy (with no false-positive and false-negative events), respectively. Thus, the AUC value of each cancer marker is likewise preferably 0.90, more preferably not less than 0.97, still more preferably not less than 0.99, and most preferably 1.00 in the present invention. The ones whose nucleotide sequences are represented by SEQ ID NOs: 162, 160, 145, 143, 146, 140, and 141 are preferable due to an AUC value of 0.97 or higher; among those, ones represented by SEQ ID NOs: 162 and 160 are more preferable due to an AUC value of 0.98 or higher.

Furthermore, because the FC (fold change) in the abundance of an isomiR represented by either SEQ ID NO: 115 or SEQ ID NO: 116 is changed before and after surgery for tongue cancer, the isomiRs can be used to assess the success or failure of the surgery.

The test sample is not specifically limited, provided that the test sample is a body fluid containing miRNAs or the like; typically, it is preferable to use a blood sample (including plasma, serum, and whole blood). For the ones or the like present in serum, it is simple and preferable to use serum or plasma as a test sample. For the miRNAs or the like present in exosomes, it is preferable to use serum or plasma as a test sample, from which exosomes are isolated to extract total RNA and to measure the abundance of each miRNA or the like. The method of extracting total RNA in serum or plasma is well known and is specifically described in Examples below. The method of extracting total RNA from exosomes in serum or plasma is itself known and is specifically described in more detail in Examples below.

The abundance of each miRNA or the like is preferably measured (quantified) using a next-generation sequencer. Any instrument may be used and is not limited to a specific type of instrument, provided that the instrument determines sequences, similarly to next-generation sequencers. In the method of the present invention, as specifically described in Examples below, use of a next-generation sequencer is preferred over quantitative reverse-transcription PCR (qRT-PCR), which is widely used for quantification of miRNAs, to perform measurements from the viewpoint of accuracy because miRNAs or the like to be quantified include, for example, isomiRs, in which only one or more nucleotides are deleted from or added to the 5' and/or 3' ends of the original mature miRNAs thereof, and which should be distinguished from the original miRNAs when measured. Briefly, though details will be described specifically in Examples below, the quantification method can be performed as follows. When the RNA content in serum or plasma is constant, among reads measured in a next-generation sequencing analysis of the RNA content, the number of reads for each isomiR or mature miRNA per million reads is considered as the measurement value, where the total counts of reads with human-derived sequences are normalized to one million reads. When the RNA content in serum or plasma is variable in comparison with healthy subjects due to a disease, miRNAs showing little abundance variation in serum and plasma may be used. In cases where the abundance of miRNAs or the like in serum or plasma is measured, at least one miRNA selected from the group consisting of let-7g-5p, miR-425-3p, and miR-425-5p is preferably used as an internal control, which are miRNAs showing little abundance variation in serum and plasma.

The cut-off value for the abundance of each miRNA or the like for use in evaluation is preferably determined based on the presence or absence of a statistically significant difference (*t*-test; *p* < 0.05, preferably *p* < 0.01, more preferably *p* < 0.001) from healthy subjects with regard to the abundance of the miRNA or the like. Specifically, the value of log₂ read counts (the cut-off value) can be preferably determined for each miRNA or the like, for example, at which the false-positive rate is optimal (the lowest); for example, the cut-off values (the values of log₂ read counts) for several miRNAs or the like are as indicated in Table 2. The cut-off values indicated in Table 2 are only examples, and other values may be employed as cut-off values as long as those values are appropriate to determine statistically significant difference. Additionally, the optimal cut-off values vary among different populations of patients and healthy subjects from which data is collected. However, the cut-off values indicated in Table 2 or 3 with an interval of usually ±20%, particularly ±10%, may be set as cut-off values.

Each of the above miRNAs or the like is statistically significantly different in abundance between patients with head and neck cancer and healthy subjects, and may thus be used alone as an index. However, a combination of multiple miRNAs or the like may also be used as an index, which can assist in more accurate detection of head and neck cancer.

Moreover, a method of detecting the abundance of miRNAs or the like in a test sample from human suspected of having or affected with head and neck cancer is also provided. That is, a method of detecting the abundance of at least one of miRNAs, isoform miRNAs (isomiRs), precursor miRNAs, transfer RNA fragments (tRFs), or non-coding RNA fragments (LincRNAs or MiscRNAs) whose nucleotide sequence is represented by any one of SEQ ID NOs: 162, 160, 145, 143, 146, 140, 141, 1 to 139, 142, 144, 147 to 159, and 161 in a test sample from human suspected of having or affected with head and neck cancer is also provided, wherein the method includes the steps of:
collecting a blood sample from human; and
measuring the abundance of the miRNA(s), isoform miRNA(s) (isomiR(s)), precursor miRNA(s), transfer RNA fragment(s) (tRF(s)), or non-coding RNA fragment(s) (LincRNA(s) or MiscRNA(s)) in the blood sample by means of a next-generation sequencer or qRT-PCR,
wherein the abundance of at least one of the miRNAs, isomiRs, precursor miRNAs, transfer RNA fragments, or non-coding RNA fragments whose nucleotide sequence is represented by any one of SEQ ID NOs: 1 to 71 and 118 to 136 is higher than that in healthy subjects, or the abundance of at least one of the miRNAs, isomiRs, precursor miRNAs, transfer RNA fragments, or non-coding RNA fragments whose nucleotide sequence is represented by any one of SEQ ID NOs: 72 to 117 and 137 to 162 is lower than that in healthy subjects.

In the present invention, the term head and neck cancer includes, for example, tongue cancer (oral cavity cancer), maxillary sinus cancer, nasopharyngeal cancer, oropharyngeal cancer, hypopharyngeal cancer, laryngeal cancer, thyroid cancer, salivary gland cancer, and metastatic cervical carcinoma from unknown primary.

Additionally, in cases where the detection of head and neck cancer is successfully achieved by the above-described method of the present invention, an effective amount of an anti-head and neck cancer drug can be administered to patients in whom head and neck cancer is detected, to treat the head and neck cancer. Examples of the anti-head and neck cancer drug can include cisplatin (CDDP), 5-FU (5-fluorouracil), and docetaxel.

The present invention will be specifically described below by way of examples and comparative examples. Naturally, the present invention is not limited by the examples below.

### Examples 1 to 165

### 1. Materials and Methods

### (1) Clinical Samples

Plasma samples from 24 patients with head and neck cancer and from 10 healthy subjects were used.

### (2) Extraction of RNA in Serum

Extraction of RNA in serum was performed using the miRNeasy Mini kit (QIAGEN).
1) Each frozen plasma sample was thawed and centrifuged at 10000 rpm for 5 minutes at room temperature to precipitate aggregated proteins and blood cell components.
2) To a new 1.5-mL tube, 200 µL of the supernatant was transferred.
3) To the tube, 1000 µL of the QIAzol Lysis Reagent was added and mixed thoroughly to denature protein components.
4) To the tube, 10 µL of 0.05 nM cel-miR-39 was added as a control RNA for RNA extraction, mixed by pipetting, and then left to stand at room temperature for 5 minutes.
5) To promote separation of the aqueous and organic solvent layers, 200 µL of chloroform was added to the tube, mixed thoroughly, and left to stand at room temperature for 3 minutes.
6) The tube was centrifuged at 12000 x g for 15 minutes at 4°C and 650 µL of the upper aqueous layer was transferred to a new 2-mL tube.
7) For the separation of RNA, 975 µL of 100% ethanol was added to the tube and mixed by pipetting.
8) To a miRNeasy Mini spin column (hereinafter referred to as column), 650 µL of the mixture in the step 7 was transferred, left to stand at room temperature for 1 minute, and then centrifuged at 8000 x g for 15 seconds at room temperature to allow RNA to be adsorbed on the filter of the column. The flow-through solution from the column was discarded.
9) The step 8 was repeated until the total volume of the solution of the step 7 was filtered through the column to allow all the RNA to be adsorbed on the filter.
10) To remove impurities attached on the filter, 650 µL of Buffer RWT was added to the column and centrifuged at 8000 x g for 15 seconds at room temperature. The flow-through solution from the column was discarded.
11) To clean the RNA adsorbed on the filter, 500 µL of Buffer RPE was added to the column and centrifuged at 8000 x g for 15 seconds at room temperature. The flow-through solution from the column was discarded.
12) To clean the RNA adsorbed on the filter, 500 µL of Buffer RPE was added to the column and centrifuged at 8000 x g for 2 minutes at room temperature. The flow-through solution from the column was discarded.
13) To completely remove any solution attached on the filter, the column was placed in a new 2-mL collection tube and centrifuged at 10000 x g for 1 minute at room temperature.
14) The column was placed into a 1.5-mL tube and 50 µl of RNase-free water was added thereto and left to stand at room temperature for 1 minute.
15) Centrifugation was performed at 8000 x g for 1 minute at room temperature to elute the RNA adsorbed on the filter. The eluted RNA was used in the following experiment without further purification and the remaining portion of the eluted RNA was stored at -80°C.

### (3) Extraction of RNA from Exosomes

Exosomes in serum were collected as follows.

Exosome isolation was performed with the Total Exosome Isolation (from serum) from Thermo Fisher Scientific, Inc. Extraction of RNA from the collected exosomes was performed using the miRNeasy Mini kit (QIAGEN).

### (4) Quantification of miRNAs or the Like

The quantification of miRNAs or the like was performed as follows.

In cases where miRNAs or the like from, for example, two groups are quantified, extracellular vesicles (including exosomes) isolated by the same method are used to purify RNAs through the same method, from which cDNA libraries are prepared and then analyzed by next-generation sequencing. The next-generation sequencing analysis is not limited by a particular instrument, provided that the instrument determines sequences.

### (5) Calculation of Cut-off Value and AUC

Specifically, the cut-off value and the AUC were calculated from measurement results as follows. The logistic regression analysis was carried out using the JMP Genomics 8 to draw the ROC curve and to calculate the AUC. Moreover, the value corresponding to a point on the ROC curve which was closest to the upper left corner of the ROC graph (sensitivity: 1.0, specificity: 1.0) was defined as the cut-off value.

### 2. Results

The results are presented in Tables 2-1 to 2-10.

**Table 2-1**

| Example | SEQ ID NO: | Class | Archetype | Type | Length (nucleotides) | Average in head and neck cancer patients | Average in healthy subjects | Log2 FC | AUC | Cut-off value (Log2) | p-value |
|---|---|---|---|---|---|---|---|---|---|---|---|
| Example 1 | 1 | tRF | tRNA-Gly-CCC-1-1/ /··· *1 | Exact | 30 | 1758 | 65 | 3.81 | 0.900 | 6.08 | 0.000 |
| Example 2 | 2 | tRF | tRNA-Lys-TTT-3-1// ... *2 | Exact | 28 | 98 | 5 | 4.57 | 0.958 | 5.18 | 0.000 |
| Example 3 | 3 | tRF | tRNA-Glu-CTC-1-1// ··· *3 | Exact | 32 | 735 | 52 | 3.67 | 0.879 | 6.59 | 0.001 |
| Example 4 | 4 | tRF | tRNA-Pro-TGG-2-1 | Exact | 31 | 106 | 8 | 4.12 | 0.883 | 4.60 | 0.000 |
| Example 5 | 5 | tRF | tRNA-Lys-TTT-3-1// ... *4 | Exact | 31 | 243 | 20 | 3.68 | 0.921 | 6.26 | 0.000 |
| Example 6 | 6 | tRF | tRNA-iMet-CAT-1-1 //··· *5 | Exact | 33 | 83 | 8 | 3.48 | 0.896 | 5.11 | 0.000 |
| Example 7 | 7 | tRF | tRNA-Lys-CTT-1-1// ... *6 | Exact | 31 | 136 | 15 | 3.14 | 0.888 | 5.00 | 0.001 |
| Example 8 | 8 | tRF | tRNA-iMet-CAT-1-1 //··· *7 | Exact | 31 | 51 | 7 | 3.48 | 0.904 | 4.15 | 0.000 |
| Example 9 | 9 | isomiR | mir-183 | Mature 5' sub | 21 | 91 | 12 | 2.32 | 0.777 | 5.16 | 0.007 |
| Example 10 | 10 | isomiR | mir-223 | Mature 3' sub | 17 | 526 | 78 | 2.96 | 0.879 | 5.95 | 0.000 |
| Example 11 | 11 | miRNA | mir-150 | Mature 5' | 22 | 17236 | 2591 | 2.39 | 0.896 | 12.74 | 0.000 |
| Example 12 | 12 | isomiR | mir-223 | Mature 3' super | 24 | 289 | 44 | 2.59 | 0.865 | 6.70 | 0.003 |
| Example 13 | 13 | tRF | tRNA-Lys-CTT-1-1// ··· *8 | Exact | 28 | 94 | 15 | 3.10 | 0.850 | 4.72 | 0.001 |
| Example 14 | 14 | isomiR | mir-150 | Mature 5' super | 23 | 80 | 13 | 3.10 | 0.875 | 5.51 | 0.000 |
| Example 15 | 15 | isomiR | mir-150 | Mature 5' sub | 19 | 337 | 60 | 3.33 | 0.846 | 7.32 | 0.008 |
| Example 16 | 16 | tRF | tRNA-Pro-AGG-1-1/ /··· *9 | Exact | 30 | 523 | 94 | 4.22 | 0.850 | 5.68 | 0.003 |
| Example 17 | 17 | isomiR | mir-146b | Mature 5' super | 23 | 191 | 35 | 2.16 | 0.873 | 5.77 | 0.005 |
| Example 18 | 18 | tRF | tRNA-iMet-CAT-1-1 //··· *10 | Exact | 30 | 125 | 22 | 3.03 | 0.931 | 5.97 | 0.000 |

**Table 2-2**

| Example | SEQ ID NO: | Class | Archetype | Type | Length (nucleotides) | Average in head and neck cancer patients | Average in healthy subjects | Log2 FC | AUC | Cut-off value (Log2) | p-value |
|---|---|---|---|---|---|---|---|---|---|---|---|
| Example 19 | 19 | isomiR | mir-361 | Mature 3' super | 24 | 35 | 7 | 2.58 | 0.850 | 4.59 | 0.001 |
| Example 20 | 20 | isomiR | mir-223 | Mature 3' sub/super | 21 | 270 | 59 | 2.56 | 0.842 | 7.16 | 0.001 |
| Example 21 | 21 | precursor | mir-223 | precursor miRNA | 15 | 293 | 67 | 2.14 | 0.821 | 5.68 | 0.005 |
| Example 22 | 22 | precursor | mir-223 | precursor miRNA | 16 | 317 | 73 | 2.71 | 0.833 | 6.67 | 0.005 |
| Example 23 | 23 | isomiR | mir-146a | Mature 5' sub | 20 | 31 | 8 | 2.37 | 0.796 | 3.61 | 0.002 |
| Example 24 | 24 | isomiR | mir-150 | Mature 5' sub | 20 | 1205 | 298 | 2.01 | 0.800 | 9.70 | 0.002 |
| Example 25 | 25 | isomiR | mir-223 | Mature 3' sub | 18 | 356 | 92 | 2.11 | 0.838 | 6.44 | 0.009 |
| Example 26 | 26 | miRNA | mir-29a | Mature 3' | 22 | 1384 | 355 | 2.23 | 0.858 | 9.40 | 0.000 |
| Example 27 | 27 | isomiR | mir-223 | Mature 3' sub | 20 | 117 | 30 | 2.31 | 0.821 | 5.23 | 0.004 |
| Example 28 | 28 | miRNA | mir-339 | Mature 5' | 23 | 39 | 10 | 2.51 | 0.796 | 3.71 | 0.002 |
| Example 29 | 29 | isomiR | mir-223 | Mature 3' super | 23 | 110411 | 30866 | 1.80 | 0.846 | 14.64 | 0.001 |
| Example 30 | 30 | miRNA | mir-146b | Mature 5' | 22 | 303 | 83 | 1.35 | 0.829 | 6.73 | 0.001 |
| Example 31 | 31 | isomiR | mir-365a//mir-365b | Mature 3' sub | 21 | 55 | 16 | 1.98 | 0.833 | 4.11 | 0.003 |
| Example 32 | 32 | miRNA | mir-140 | Mature 5' | 22 | 172 | 49 | 2.15 | 0.938 | 6.41 | 0.006 |
| Example 33 | 33 | miRNA | mir-223 | Mature 3' | 22 | 78031 | 24601 | 1.57 | 0.825 | 15.54 | 0.002 |
| Example 34 | 34 | isomiR | mir-223 | Mature 3' sub/super | 22 | 24932 | 7946 | 1.73 | 0.821 | 12.89 | 0.001 |
| Example 35 | 35 | tRF | tRNA-Leu-AAG-1-1/ /··· *11 | Exact | 16 | 134 | 42 | 1.68 | 0.546 | 7.34 | 0.041 |
| Example 36 | 36 | isomiR | mir-150 | Mature 5' sub | 21 | 7252 | 2372 | 1.61 | 0.738 | 11.13 | 0.023 |
| Example 37 | 37 | isomiR | mir-146b | Mature 5' super | 24 | 255 | 85 | 1.53 | 0.850 | 6.54 | 0.001 |
| Example 38 | 38 | tRF | tRNA-Glu-CTC-1-1// ... *12 | Exact | 30 | 86 | 28 | 1.63 | 0.771 | 5.99 | 0.001 |
| Example 39 | 39 | isomiR | mir-223 | Mature 3' sub | 20 | 2960 | 1043 | 1.86 | 0.792 | 8.85 | 0.002 |
| Example 40 | 40 | isomiR | mir-145 | Mature 5' super | 24 | 116 | 41 | 1.50 | 0.790 | 5.48 | 0.005 |
| Example 41 | 41 | isomiR | mir-186 | Mature 5' sub | 21 | 322 | 112 | 1.53 | 0.921 | 7.74 | 0.000 |

**Table 2-3**

| Example | SEQ ID NO: | Class | Archetype | Type | Length (nucleotides) | Average in head and neck cancer patients | Average in healthy subjects | Log2 FC | AUC | Cut-off value (Log2) | p-value |
|---|---|---|---|---|---|---|---|---|---|---|---|
| Example 42 | 42 | miRNA | mir-365a//mir-365b | Mature 3' | 22 | 169 | 61 | 1.29 | 0.808 | 6.55 | 0.005 |
| Example 43 | 43 | isomiR | mir-223 | Mature 3' super | 23 | 167 | 62 | 1.43 | 0.700 | 6.90 | 0.012 |
| Example 44 | 44 | isomiR | mir-192 | Mature 5' sub | 20 | 344 | 130 | 1.40 | 0.608 | 7.93 | 0.033 |
| Example 45 | 45 | tRF | tRNA-Gly-GCC-2-1/ /··· *13 | Exact | 33 | 131 | 50 | 1.38 | 0.733 | 4.10 | 0.047 |
| Example 46 | 46 | miRNA | mir-17 | Mature 5' | 23 | 1458 | 590 | 1.39 | 0.888 | 9.88 | 0.000 |
| Example 47 | 47 | isomiR | mir-339 | Mature 5' sub | 19 | 156 | 64 | 1.29 | 0.748 | 5.61 | 0.011 |
| Example 48 | 48 | isomiR | mir-223 | Mature 3' sub | 21 | 6065 | 2585 | 1.23 | 0.763 | 11.58 | 0.007 |
| Example 49 | 49 | isomiR | mir-223 | Mature 3' sub | 21 | 10177 | 4407 | 1.21 | 0.754 | 11.30 | 0.010 |
| Example 50 | 50 | isomiR | mir-30c-2//mir-30c-1 | Mature 5' sub | 22 | 86 | 36 | 1.26 | 0.754 | 5.77 | 0.007 |
| Example 51 | 51 | isomiR | mir-1307 | Mature 3' super | 23 | 46 | 20 | 1.18 | 0.767 | 5.33 | 0.003 |
| Example 52 | 52 | miRNA | mir-29c | Mature 3' | 22 | 704 | 310 | 1.50 | 0.796 | 8.76 | 0.002 |
| Example 53 | 53 | isomiR | mir-223 | Mature 3' sub | 20 | 517 | 232 | 1.16 | 0.738 | 6.16 | 0.016 |
| Example 54 | 54 | isomiR | mir-223 | Mature 3' super | 24 | 94 | 42 | 1.17 | 0.617 | 6.32 | 0.047 |
| Example 55 | 55 | isomiR | mir-30b | Mature 5' sub | 21 | 93 | 41 | 1.19 | 0.742 | 6.27 | 0.008 |
| Example 56 | 56 | isomiR | mir-766 | Mature 3' sub | 21 | 78 | 36 | 1.11 | 0.733 | 5.34 | 0.012 |
| Example 57 | 57 | isomiR | mir-26b | Mature 3' sub | 21 | 37 | 17 | 1.11 | 0.744 | 4.02 | 0.017 |
| Example 58 | 58 | tRF | tRNA-Gly-CCC-1-1/ / ··· *14 | Exact | 22 | 310 | 140 | 1.14 | 0.631 | 9.06 | 0.037 |
| Example 59 | 59 | miRNA | let-7d | Mature 3' | 22 | 103 | 48 | 1.12 | 0.802 | 6.86 | 0.003 |
| Example 60 | 60 | tRF | tRNA-Gly-CCC-1-1/ /··· *15 | Exact | 25 | 415 | 191 | 1.12 | 0.617 | 9.15 | 0.053 |
| Example 61 | 61 | isomiR | mir-30d | Mature 5' sub | 19 | 144 | 69 | 1.07 | 0.721 | 6.82 | 0.016 |
| Example 62 | 62 | miRNA | mir-505 | Mature 3' | 22 | 55 | 26 | 1.08 | 0.767 | 5.34 | 0.007 |
| Example 63 | 63 | isomiR | mir-93 | Mature 5' sub | 22 | 61 | 28 | 1.13 | 0.767 | 4.66 | 0.032 |
| Example 64 | 64 | isomiR | mir-30e | Mature 5' super | 23 | 817 | 384 | 1.09 | 0.867 | 9.44 | 0.000 |

**Table 2-4**

| Example | SEQ ID NO: | Class | Archetype | Type | Length (nucleotides) | Average in head and neck cancer patients | Average in healthy subjects | Log2 FC | AUC | Cut-off value (Log2) | p-value |
|---|---|---|---|---|---|---|---|---|---|---|---|
| Example 65 | 65 | precursor | mir-16-1//mir-16-2 | precursor miRNA | 16 | 114 | 54 | 1.09 | 0.740 | 6.33 | 0.012 |
| Example 66 | 66 | miRNA | mir-193a | Mature 5' | 22 | 245 | 121 | 1.19 | 0.771 | 7.30 | 0.006 |
| Example 67 | 67 | isomiR | mir-320a | Mature 3' super | 25 | 46 | 22 | 1.07 | 0.717 | 4.37 | 0.019 |
| Example 68 | 68 | isomiR | mir-29b-1//mir-29b-2 | Mature 3' sub | 21 | 187 | 93 | 1.01 | 0.650 | 7.06 | 0.023 |
| Example 69 | 69 | isomiR | mir-142 | Mature 5' sub/super | 22 | 458 | 242 | 0.92 | 0.717 | 8.13 | 0.043 |
| Example 70 | 70 | isomiR | mir-142 | Mature 5' sub/super | 21 | 117 | 60 | 0.97 | 0.73 1 | 5.33 | 0.045 |
| Example 71 | 71 | miRNA | mir-744 | Mature 5' | 22 | 131 | 69 | 0.92 | 0.758 | 6.31 | 0.012 |
| Example 72 | 72 | isomiR | mir-200b | Mature 3' sub | 21 | 2 | 27 | -3.48 | 0.900 | 2.69 | 0.000 |
| Example 73 | 73 | isomiR | mir-181b-1//mir-181 b-2 | Mature 5' sub | 19 | 20 | 203 | -5.29 | 0.946 | 5.09 | 0.000 |
| Example 74 | 74 | isomiR | mir-200a | Mature 3' sub | 18 | 5 | 47 | -4.05 | 0.950 | 4.13 | 0.000 |
| Example 75 | 75 | isomiR | mir-181b-1//mir-181 b-2 | Mature 5' sub | 18 | 37 | 296 | -5.43 | 0.942 | 5.40 | 0.000 |
| Example 76 | 76 | isomiR | mir-181b-1//mir-181 b-2 | Mature 5' sub | 20 | 79 | 583 | -5.95 | 0.917 | 5.40 | 0.000 |
| Example 77 | 77 | miRNA | mir-340 | Mature 3' | 22 | 312 | 2209 | -7.02 | 0.938 | 8.82 | 0.000 |
| Example 78 | 78 | isomiR | mir-181b-1//mir-181 b-2 | Mature 5' sub | 21 | 33 | 223 | -4.97 | 0.921 | 5.40 | 0.000 |
| Example 79 | 79 | miRNA | mir-378e | Mature 3' | 19 | 5 | 33 | -3.37 | 0.865 | 2.69 | 0.000 |
| Example 80 | 80 | precursor | mir-181b-1//mir-181 b-2 | precursor miRNA | 17 | 17 | 100 | -4.43 | 0.925 | 5.80 | 0.000 |
| Example 81 | 81 | isomiR | mir-145 | Mature 5' sub | 19 | 6 | 32 | -3.42 | 0.867 | 3.21 | 0.000 |
| Example 82 | 82 | precursor | mir-181b-1//mir-181 b-2 | precursor miRNA | 16 | 12 | 71 | -3.96 | 0.873 | 4.61 | 0.000 |

Table 2-5

| Example | SEQ ID NO: | Class | Archetype | Type | Length (nucleotides) | Average in head and neck cancer patients | Average in healthy subjects | Log2 FC | AUC | Cut-off value (Log2) | p-value |
|---|---|---|---|---|---|---|---|---|---|---|---|
| Example 83 | 83 | isomiR | mir-181b-1//mir-181 b-2 | Mature 5' sub | 22 | 64 | 343 | -4.91 | 0.925 | 6.37 | 0.000 |
| Example 84 | 84 | isomiR | mir-451a | Mature 5' sub | 18 | 7 | 33 | -3.31 | 0.942 | 3.81 | 0.000 |
| Example 85 | 85 | isomiR | mir-29b-1//mir-29b-2 | Mature 3' sub | 22 | 15 | 69 | -3.75 | 0.863 | 2.69 | 0.000 |
| Example 86 | 86 | isomiR | mir-451a | Mature 5' sub | 17 | 13 | 55 | -2.90 | 0.913 | 4.67 | 0.000 |
| Example 87 | 87 | precursor | mir-181b-1//mir-181 b-2 | precursor miRNA | 15 | 9 | 38 | -3.16 | 0.844 | 4.63 | 0.000 |
| Example 88 | 88 | isomiR | mir-144 | Mature 3' sub | 17 | 20 | 75 | -2.55 | 0.854 | 5.64 | 0.002 |
| Example 89 | 89 | isomiR | mir-451a | Mature 5' sub/super | 18 | 16 | 55 | -2.15 | 0.850 | 5.48 | 0.009 |
| Example 90 | 90 | isomiR | mir-451a | Mature 5' sub | 19 | 14 | 46 | -2.46 | 0.850 | 4.58 | 0.000 |
| Example 91 | 91 | miRNA | let-7e | Mature 5' | 22 | 11 | 35 | -2.24 | 0.821 | 3.18 | 0.002 |
| Example 92 | 92 | isomiR | mir-16-2 | Mature 3' sub/super | 20 | 119 | 362 | -1.87 | 0.967 | 7.97 | 0.000 |
| Example 93 | 93 | isomiR | mir-451a | Mature 5' super | 25 | 11282 | 31795 | -1.49 | 0.671 | 14.65 | 0.043 |
| Example 94 | 94 | isomiR | mir-486-1 | Mature 5' super | 23 | 15 | 42 | -1.48 | 0.796 | 4.18 | 0.020 |
| Example 95 | 95 | isomiR | mir-126 | Mature 3' sub | 20 | 29 | 80 | -1.87 | 0.842 | 5.55 | 0.006 |
| Example 96 | 96 | isomiR | mir-363 | Mature 3' sub | 19 | 15 | 38 | -1.39 | 0.802 | 3.98 | 0.022 |
| Example 97 | 97 | isomiR | mir-574 | Mature 5' sub | 21 | 22 | 56 | -2.16 | 0.829 | 5.18 | 0.001 |
| Example 98 | 98 | miRNA | let-7b | Mature 5' | 22 | 1771 | 4518 | -1.28 | 0.817 | 10.67 | 0.001 |
| Example 99 | 99 | miRNA | mir-144 | Mature 3' | 20 | 660 | 1687 | -1.35 | 0.771 | 9.97 | 0.028 |
| Example 100 | 100 | isomiR | mir-574 | Mature 3' sub | 21 | 17 | 43 | -2.04 | 0.846 | 4.22 | 0.000 |
| Example 101 | 101 | isomiR | let-7b | Mature 5' sub | 21 | 1614 | 3915 | -1.50 | 0.900 | 10.98 | 0.000 |
| Example 102 | 102 | isomiR | mir-103a-2//mir-103a -1//mir-107 | Mature 3' sub | 19 | 648 | 1544 | -1.06 | 0.717 | 10.94 | 0.008 |
| Example 103 | 103 | isomiR | mir-126 | Mature 3' sub | 21 | 301 | 713 | -1.56 | 0.854 | 8.66 | 0.002 |
| Example 104 | 104 | isomiR | mir-451 a | Mature 5' super | 24 | 19 | 43 | -1.18 | 0.738 | 4.01 | 0.072 |
| Example 105 | 105 | miRNA | mir-106b | Mature 5' | 21 | 670 | 1524 | -1.13 | 0.888 | 10.36 | 0.001 |

**Table 2-6**

| Example | SEQ ID NO: | Class | Archetype | Type | Length (nucleotides) | Average in head and neck cancer patients | Average in healthy subjects | Log2 FC | AUC | Cut-off value(L og2) | p-value |
|---|---|---|---|---|---|---|---|---|---|---|---|
| Example 106 | 106 | miRNA | let-7i | Mature 5' | 22 | 107 | 247 | -1.20 | 0.804 | 7.46 | 0.014 |
| Example 107 | 107 | precursor | mir-451a | precursor miRNA | 15 | 49 | 106 | -1.11 | 0.783 | 6.13 | 0.036 |
| Example 108 | 108 | isomiR | mir-425 | Mature 5' sub | 19 | 14 | 31 | -1.13 | 0.819 | 4.10 | 0.031 |
| Example 109 | 109 | isomiR | mir-16-2 | Mature 3' sub | 20 | 15 | 33 | -1.82 | 0.754 | 4.51 | 0.003 |
| Example 110 | 110 | miRNA | mir-139 | Mature 5' | 23 | 69 | 155 | -1.18 | 0.771 | 7.08 | 0.024 |
| Example 111 | 111 | isomiR | mir-451a | Mature 5' super | 23 | 38 | 80 | -1.10 | 0.715 | 6.35 | 0.047 |
| Example 112 | 112 | isomiR | mir-18a | Mature 5' sub | 21 | 138 | 296 | -1.10 | 0.767 | 7.79 | 0.030 |
| Example 113 | 113 | miRNA | mir-126 | Mature 3' | 22 | 335 | 706 | -1.23 | 0.833 | 8.69 | 0.004 |
| Example 114 | 114 | isomiR | mir-550a-1//mir-550a -2//mir-550a-3 | Mature 3' sub | 21 | 63 | 133 | -1.50 | 0.775 | 6.23 | 0.005 |
| Example 115 | 115 | isomiR | mir-142 | Mature 3' sub | 22 | 181 | 222 | -0.30 | 0.504 | 8.05 | 0.548 |
| Example 116 | 116 | isomiR | mir-142 | Mature 3' sub | 21 | 156 | 135 | 0.21 | 0.517 | 5.74 | 0.577 |
| Example 122 | 119 | MiscRNA | ENST00000363745. 1//··· *16 | Exact | 28 | 484 | 40 | 6.44 | 0.936 | 5.79 | 0.000 |
| Example 123 | 120 | MiscRNA | ENST00000364600. 11/··· *17 | Exact | 31 | 1504 | 95 | 6.35 | 0.951 | 8.41 | 0.000 |
| Example 124 | 121 | miRNA | mir-221 | Mature 3' | 23 | 457 | 32 | 5.92 | 0.923 | 7.09 | 0.000 |
| Example 125 | 122 | miRNA | mir-374b | Mature 5' | 22 | 465 | 44 | 5.44 | 0.931 | 7.50 | 0.000 |
| Example 126 | 123 | isomiR | mir-130a | Mature 3' super | 23 | 293 | 32 | 5.43 | 0.904 | 6.27 | 0.000 |
| Example 127 | 124 | miRNA | mir-340 | Mature 5' | 22 | 495 | 47 | 5.40 | 0.932 | 7.23 | 0.000 |
| Example 128 | 125 | miRNA | mir-199a-1//mir-199a -2//mir-199b | Mature 3' | 22 | 2387 | 161 | 5.21 | 0.958 | 9.23 | 0.000 |
| Example 129 | 126 | isomiR | mir-23a | Mature 3' super | 23 | 927 | 92 | 4.98 | 0.914 | 8.22 | 0.000 |
| Example 130 | 127 | miRNA | mir-335 | Mature 5' | 23 | 632 | 89 | 4.84 | 0.949 | 7.50 | 0.000 |
| Example 131 | 128 | miRNA | mir-130a | Mature 3' | 22 | 3873 | 417 | 3.70 | 0.962 | 10.40 | 0.000 |
| Example 132 | 129 | isomiR | mir-584 | Mature 5' sub | 21 | 619 | 121 | 3.38 | 0.897 | 8.04 | 0.000 |
| Example 133 | 130 | MiscRNA | ENST00000363745. 1//··· *18 | Exact | 26 | 13226 | 2207 | 2.72 | 0.908 | 12.82 | 0.000 |

**Table 2-7**

| Example | SEQ ID NO: | Class | Archetype | Type | Length (nucleotides) | Average in head and neck cancer patients | Average in healthy subjects | Log2 FC | AUC | Cut-off value (Log2) | p-value |
|---|---|---|---|---|---|---|---|---|---|---|---|
| Example 134 | 131 | miRNA | mir-26a-1//mir-26a-2 | Mature 5' | 22 | 5509 | 853 | 2.66 | 0.931 | 11.03 | 0.000 |
| Example 135 | 132 | MiscRNA | ENST00000364600. 1//··· *17 | Exact | 32 | 151813 | 17667 | 2.56 | 0.932 | 15.67 | 0.000 |
| Example 136 | 133 | isomiR | mir-23a | Mature 3' super | 22 | 12447 | 2197 | 2.19 | 0.947 | 12.60 | 0.000 |
| Example 137 | 134 | miRNA | mir-146a | Mature 5' | 22 | 2236 | 549 | 2.05 | 0.915 | 10.03 | 0.000 |
| Example 138 | 135 | miRNA | mir-191 | Mature 5' | 23 | 3434 | 726 | 2.04 | 0.926 | 10.19 | 0.000 |
| Example 139 | 136 | MiscRNA | ENST00000364600. 1//··· *17 | Exact | 31 | 106642 | 25718 | 2.02 | 0.939 | 15.70 | 0.000 |
| Example 140 | 137 | miRNA | mir-92a-1//mir-92a-2 | Mature 3' | 22 | 2418 | 8103 | -2.07 | 0.941 | 11.90 | 0.000 |
| Example 141 | 138 | isomiR | let-7b | Mature 5' sub | 20 | 416 | 1273 | -2.15 | 0.901 | 9.56 | 0.000 |
| Example 142 | 139 | isomiR | mir-451 a | Mature 5' sub | 21 | 13722 | 36210 | -2.15 | 0.905 | 14.34 | 0.000 |
| Example 143 | 140 | isomiR | mir-30e | Mature 5' sub/super | 23 | 414 | 1361 | -2.21 | 0.972 | 9.67 | 0.000 |
| Example 144 | 141 | isomiR | let-7g | Mature 5' sub | 21 | 875 | 3513 | -2.28 | 0.972 | 10.48 | 0.000 |
| Example 145 | 142 | miRNA | mir-486-1//mir-486-2 | Mature 5' | 22 | 2037 | 7408 | -2.44 | 0.935 | 11.36 | 0.000 |
| Example 146 | 143 | isomiR | mir-16-1//mir-16-2 | Mature 5' sub | 20 | 2087 | 8031 | -2.47 | 0.977 | 12.12 | 0.000 |
| Example 147 | 144 | isomiR | mir-451a | Mature 5' sub | 20 | 7902 | 30578 | -2.61 | 0.957 | 14.22 | 0.000 |
| Example 148 | 145 | isomiR | mir-185 | Mature 5' sub | 21 | 595 | 2886 | -2.67 | 0.978 | 10.52 | 0.000 |
| Example 149 | 146 | isomiR | let-7a-1//let-7a-2//let-7a-3 | Mature 5' sub | 20 | 633 | 3159 | -2.67 | 0.975 | 10.97 | 0.000 |
| Example 150 | 147 | isomiR | mir-92a-1//mir-92a-2 | Mature 3' sub | 21 | 247 | 882 | -2.73 | 0.904 | 8.30 | 0.000 |
| Example 151 | 148 | isomiR | mir-25 | Mature 3' sub | 21 | 214 | 916 | -2.86 | 0.961 | 8.79 | 0.000 |
| Example 152 | 149 | isomiR | mir-16-2 | Mature 3' sub/super | 21 | 159 | 708 | -2.87 | 0.921 | 8.60 | 0.000 |

**Table 2-8**

| Example | SEQ ID NO: | Class | Archetype | Type | Length (nucleotides) | Average in head and neck cancer patients | Average in healthy subjects | Log2 FC | AUC | Cut-off value (Log2) | p-value |
|---|---|---|---|---|---|---|---|---|---|---|---|
| Example 153 | 150 | isomiR | let-7f-1//let-7f-2 | Mature 5' sub | 20 | 253 | 1372 | -2.98 | 0.956 | 9.04 | 0.000 |
| Example 154 | 151 | isomiR | mir-25 | Mature 3' sub | 20 | 117 | 538 | -3.01 | 0.931 | 7.93 | 0.000 |
| Example 155 | 152 | isomiR | mir-425 | Mature 5' sub | 21 | 147 | 634 | -3.15 | 0.945 | 8.53 | 0.000 |
| Example 156 | 153 | isomiR | mir-423 | Mature 5' sub | 21 | 588 | 2940 | -3.15 | 0.962 | 10.52 | 0.000 |
| Example 157 | 154 | isomiR | mir-484 | Mature 5' sub | 21 | 635 | 3996 | -3.27 | 0.966 | 10.23 | 0.000 |
| Example 158 | 155 | isomiR | mir-486-1//mir-486-2 | Mature 5' sub | 21 | 2876 | 17383 | -3.32 | 0.956 | 12.95 | 0.000 |
| Example 159 | 156 | isomiR | mir-486-1//mir-486-2 | Mature 5' sub | 20 | 280 | 1771 | -3.48 | 0.952 | 9.47 | 0.000 |
| Example 160 | 157 | isomiR | let-7i | Mature 5' sub | 21 | 460 | 3333 | -3.61 | 0.969 | 10.35 | 0.000 |
| Example 161 | 158 | isomiR | let-7d | Mature 5' sub | 20 | 116 | 685 | -3.75 | 0.943 | 8.46 | 0.000 |
| Example 162 | 159 | isomiR | mir-486-1//mir-486-2 | Mature 5' sub | 17 | 20 | 207 | -4.08 | 0.917 | 6.00 | 0.000 |
| Example 163 | 160 | isomiR | let-7i | Mature 5' sub | 20 | 89 | 857 | -4.36 | 0.981 | 8.54 | 0.000 |
| Example 164 | 161 | isomiR | mir-484 | Mature 5' sub | 20 | 43 | 497 | -4.85 | 0.964 | 7.76 | 0.000 |
| Example 165 | 162 | LincRNA | ENST00000627566. 1 | Exact | 15 | 8 | 349 | -7.39 | 0.986 | 3.97 | 0.000 |
| Example 167 | 117 | miRNA | mir-339 | Mature 3' | 23 | 4 | 8 | 0.55 | 0.625 | 11.4 | 0.413 |
| Example 168 | 118 | miRNA | mir-17 | Mature 3' | 22 | 17 | 8 | -0.96 | 0.621 | 17.17 | 0.250 |

**Table 2-9**

| Example | SEQ ID NOs: | Class | Archetype and Type | Fold Change |
|---|---|---|---|---|
| Example 117 | 115, 116 | isomiRNA | mir-142 Mature 3' sub | Before surgery: -2.1 |
| | | | | After surgery: -2.4 |

**Table 2-10**

| Examples | SEQ ID NOs: | Class | Archetype and Type | Cut-off value | AUC value |
|---|---|---|---|---|---|
| Example 118 | 11 and 30 | miRNA | mir-150-5p and mir-146b-5p | 4.83 | 0.97628 |
| Example 119 | 11 and 26 | miRNA | mir-150-5p and mir-29a-3p | 5.05 | 0.96443 |
| Example 120 | 11 and 117 | miRNA | mir-150-5p and mir-339-3p | 4.82 | 0.94071 |
| Example 121 | 30 and 118 | miRNA | mir-146b-5p and mir-17-3p | 5.05 | 0.91406 |
| Example 166 | 157 and 162 | isomiR, LincRNA | let-7i Mature 5' sub and ENST00000627566.1 | 3.03 | 0.967 |

As seen in these results, the abundance of the miRNAs or the like represented by SEQ ID NOs: 1 to 71 and 118 to 136 was significantly higher in the patients with head and neck cancer than that in the healthy subjects, and the miRNAs or the like represented by SEQ ID NOs: 72 to 117 and 137 to 162 was significantly lower in the patients with head and neck cancer than in the healthy subjects. It was indicated that head and neck cancer was able to be detected with high accuracy by the method of the present invention (Examples 1 to 116, 122 to 165, and 167 to 168).

Moreover, the result presented in Table 2-9 showed that the FC (fold change) in the abundance of the isomiR represented by either SEQ ID NO: 115 or SEQ ID NO: 116 was changed before and after surgery for tongue cancer, indicating that the isomiRs can be used to assess the success or failure of the surgery. Furthermore, the result presented in Table 2-10 showed that the combinations of the miRNAs represented by SEQ ID NOs: 11 and 30, SEQ ID NOs: 11 and 26, SEQ ID NOs: 11 and 117, SEQ ID NOs: 30 and 118, and SEQ ID NOs: 157 and 162 had an AUC value ranging from 0.91406 to 0.97628, indicating that even early tongue cancer can be detected by using any of the combinations.

## Claims

1. A method of assisting the detection of head and neck cancer, using as an index the abundance of at least one of miRNAs, isoform miRNAs (isomiRs), precursor miRNAs, transfer RNA fragments (tRFs), or non-coding RNA fragments (LincRNAs or MiscRNAs) contained in a test sample isolated from a living body, whose nucleotide sequence is represented by any one of SEQ ID NOs: 162, 160, 145, 143, 146, 140, 141, 1 to 139, 142, 144, 147 to 159, and 161, wherein a higher abundance of at least one of the miRNAs, isomiRs, precursor miRNAs, transfer RNA fragments, or non-coding RNA fragments whose nucleotide sequence is represented by any one of SEQ ID NOs: 1 to 71 and 118 to 136 than that of healthy subjects or a lower abundance of at least one of the miRNAs, isomiRs, precursor miRNAs, transfer RNA fragments, or non-coding RNA fragments whose nucleotide sequence is represented by any one of SEQ ID NOs: 72 to 117 and 137 to 162 than that of healthy subjects indicates a higher likelihood of having head and neck cancer.

2. The method according to claim 1, wherein the abundance of at least one of miRNAs, isoform miRNAs (isomiRs), precursor miRNAs, transfer RNA fragments (tRFs), or non-coding RNA fragments (LincRNAs or MiscRNAs) whose nucleotide sequence is represented by any one of SEQ ID NOs: 162, 160, 145, 143, 146, 140, and 141 is used as an index.

3. The method according to claim 1, wherein the abundance of at least one of miRNAs, isoform miRNAs (isomiRs), precursor miRNAs, or transfer RNA fragments whose nucleotide sequence is represented by any one of SEQ ID NOs: 92, 2, 74, 73, 75, 84, 32, 77, 18, 1, 3 to 31, 33 to 72, 76, 78 to 83, 85 to 91, and 93 to 116 is used as an index.

4. The method according to claim 3, wherein the abundance of an isomiR whose nucleotide sequence is represented by SEQ ID NO: 115 or 116 is used as an index.

5. The method according to claim 3 or 4, wherein the abundance of a miRNA whose nucleotide sequence is represented by SEQ ID NO: 11 and a miRNA whose nucleotide sequence is represented by SEQ ID NO: 30 is used as an index.

6. The method according to claim 3 or 4, wherein the abundance of a miRNA whose nucleotide sequence is represented by SEQ ID NO: 11 and a miRNA whose nucleotide sequence is represented by SEQ ID NO: 26 is used as an index.

7. The method according to claim 3 or 4, wherein the abundance of a miRNA whose nucleotide sequence is represented by SEQ ID NO: 11 and a miRNA whose nucleotide sequence is represented by SEQ ID NO: 117 is used as an index.

8. The method according to claim 3 or 4, wherein the abundance of a miRNA whose nucleotide sequence is represented by SEQ ID NO: 30 and a miRNA whose nucleotide sequence is represented by SEQ ID NO: 118 is used as an index.

9. The method according to claim 1, wherein the abundance of a miRNA whose nucleotide sequence is represented by SEQ ID NO: 157 and a miRNA whose nucleotide sequence is represented by SEQ ID NO: 162 is used as an index.

10. The method according to any one of claims 3 to 8, wherein the head and neck cancer is tongue cancer.
